# EUROPEAN PATENT APPLICATION

(11) **EP 4 332 099 A1**
(43) Date of publication of application: **06.03.2024**
(21) Application number: 22794757.9
(22) Date of filing: 22.04.2022
(51) Int. Cl.: C07D 401/12, C07D 401/14, C07D 403/14, C07D 249/04, A61K 31/4439, A61K 31/4545, A61K 31/4192, A61P 35/00

(54) **NOVEL BIPHENYL DERIVATIVE, PREPARATION METHOD THEREFOR AND MEDICINAL USE THEREOF**

(30) Priority: 30.04.2021 CN 202110484958; 05.07.2021 CN 202110758087
(71) Applicant: Xi'an Xintong Pharmaceutical Research Co., Ltd., Xi'an, Shaanxi 710000 (CN)
(72) Inventor: XU, Yungen, Shaanxi 710000 (CN); ZHANG, Hongbo, Shaanxi 710000 (CN); ZHU, Qihua, Shaanxi 710000 (CN); DU, Huijie, Shaanxi 710000 (CN); XIA, Yu, Shaanxi 710000 (CN); YU, Chunqiu, Shaanxi 710000 (CN); HUANG, Shihui, Shaanxi 710000 (CN); LI, Hui, Shaanxi 710000 (CN); ZOU, Yi, Shaanxi 710000 (CN)
(74) Representative: Murgitroyd & Company
(86) International application number: PCT/CN2022/088339
(87) International publication number: WO 2022/228286

(57) **Abstract**

The invention relates to the field of medicinal chemistry, and discloses a class of biphenyl derivatives with PD-1/PD-L1 inhibitory activity, and a preparation method and use thereof. The invention further discloses a composition containing the biphenyl derivatives with PD-1/PD-L1 inhibitory activity or pharmaceutically acceptable salts thereof and a pharmaceutically acceptable carrier, and applications of the composition in preparation of PD-1/PD-L1 inhibitors. The compounds of the invention are useful in the therapy used to treat tumors.

## Description

### Technical field

The invention belongs to the field of medicinal chemistry, which specifically involves a class of biphenyl derivatives with PD-1/PD-L1 inhibitory activity, a preparation method, a pharmaceutical composition containing the compounds and a use of the composition in treating tumors.

### Technical background

In recent years, tumor immunotherapy has become a focus point in the field of tumor treatment. Unlike traditional treatments that directly target tumor cells, tumor immunotherapy leverages the body's own immune system to kill tumor cells. The activation of the immune checkpoint pathway will inhibit the activation of T cells, and prevent the excessive activation of the human immune system, maintain the immune tolerance of the normal body, and avoid the occurrence of autoimmune diseases. Tumors lead to tumor immune escape by over-activating immune checkpoint pathways by themselves and some lymphocytes. Among these immune checkpoints, the over-activation of PD-1/PD-L1 plays a crucial role in the development of tumors. Blocking the PD-1/PD-L1 interaction can reactivate the immune system, kill the tumor cells, and shows good efficacy in the clinical treatment of melanoma, colon cancer, non-small cell lung cancer and other tumors (Clinical and Translational Oncology, 2019, 21:702-712; The Oncologist, 2019, 24(Suppl 1):S31-S41; Human Vaccines & Immunotherapeutics,2014,10(11):3111-6; Journal of Medicinal Chemistry, 2020, 63(22):13825-13850).

Since 2014, a variety of PD-1 or PD-L1 monoclonal antibody drugs have been approved for marketing, and these monoclonal antibody drugs have made breakthroughs in the clinical treatment of various tumors. The survival cycle of many tumor patients was significantly prolonged while some patients achieved complete remission. Although the clinical effects of monoclonal antibody drugs are significant, due to the long half-life of monoclonal antibody drugs and the long binding time with the target, these drugs will cause serious immune-related adverse reactions. The production process of monoclonal antibody drugs is complicated and expensive; in addition, storage and transportation of the drugs is inconvenient. Ordinary patients can only look at the "drug" with frustration. Compared with monoclonal antibodies, small molecule drugs are inexpensive, can be administered orally, can cross biological barriers, and are easy to transport and store, offer good membrane permeability, and are non-immunogenic as well. International application WO2015034820A disclosed a small molecule inhibitor of PD-1/PD-L1, but the research on the group derived from its B ring is limited. In the absence of prior art inspiration, the inventors unexpectedly developed novel biphenyl derivatives with PD-1/PD-L1 inhibitory activity, including the derivatives of 1,2,3-triazole heterocycles.

### Scope of the Invention

The invention provides the biphenyl derivatives with PD-1/PD-L1 inhibitory activity, preparation methods therefor, and pharmaceutical applications of the derivatives as PD-1/PD-L1 inhibitors.

In the invention, PD-1/PD-L1 inhibition refers to reduction or blockade of interaction between PD-1 and PD-L1. For example, the prior art or the method set out in Example 22 of the invention can be used to quantitatively determine an inhibitory effect of the compounds on PD-1/PD-L1.

Specifically, the invention provides a biphenyl derivative or a pharmaceutically acceptable salt thereof represented by general formula (I): 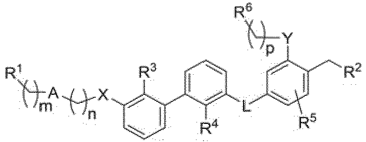 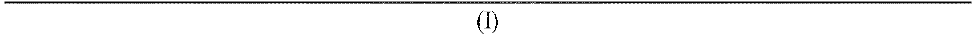 where X and Y represent O, NH, S or CH₂ respectively; *m*=0, 1 or 2; *n*=1*,* 2, 3 or 4; *p*=0 or 1; A represents a substituted phenyl or aromatic heterocyclic group, the aromatic heterocyclic group as specified is a five-membered or six-membered aromatic ring containing 1 to 3 O atoms, N or S atoms, and the substituents are H, F, Cl, Br, CN, NH₂, OH, CF₃, OCF₃, C₁-C₃ alkyl or C₁-C₃ alkoxy; L represents -CH₂O-, -CH₂NH-,
- CONH-, -NHCO-, -OCH₂-, -NH- or -CH=CH-; R¹ and R² each represent NR⁷R⁸, OR⁸ or substituted C₄-C₆ azacycloalkyl; R⁷ represents hydrogen or C₁-C₃ alkyl; R⁸ represents a substituted alkyl group from C₁-C₆, and the substituents are OH, NH₂, COOH, amido, ester, and alkoxy, which may be mono- or poly-substituted; the substituted C₄-C₆ azacycloalkyl is substituted tetrahydropyrrol-1-yl, piperidin-1-yl, morpholin-1-yl, piperazin-1-yl or azetidin-1-yl, wherein the substituents are OH, NH₂, COOH, amido, ester, alkoxy, which can be mono- or poly-substituted; R³ and R⁴ each represents H, F, Cl, Br, CN, CF₃, C₁-C₃ alkyl or cyclopropyl; R⁵ represents H, F, Cl, Br, CN, NH₂, OH, CF₃, OCF₃, C₁-C₃ alkyl or C₁-C₃ alkoxy; and R⁶ represents H, C₁-C₃ alkyl, C₁-C₃ haloalkyl, C₃-C₆ cycloalkyl, substituted phenyl or substituted aromatic heterocyclic group, and the aromatic heterocyclic group as specified contains the five-membered or six-membered aromatic ring with 1-3 O, N or S atoms, and the substituents are H, F, Cl, Br, CN, amido, sulfonamide, methanesulfonyl, and/or trifluoromethyl.

The biphenyl derivatives represented by the general formula (I) of the invention are preferably 1,2,3-triazole heterocyclic derivatives, where X and Y are preferably O; *m* is preferably 1, *n* is preferably 2 or 3, and *p* is preferably 1; A is preferably a 1,4-substituted 1,2,3-triazole rings; and L is preferably -CH₂O-.
R¹ and R² preferably represent: 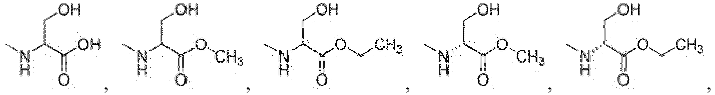 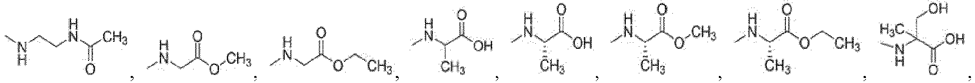 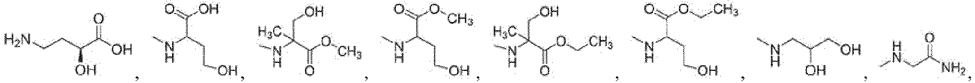 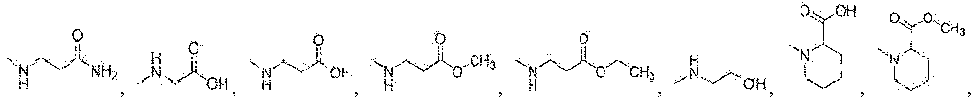 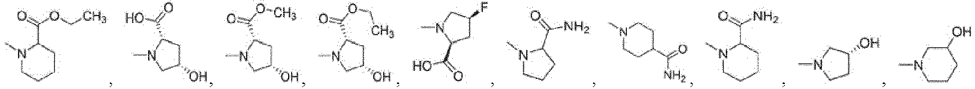 or 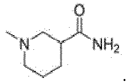
R¹ and R² more preferably represent 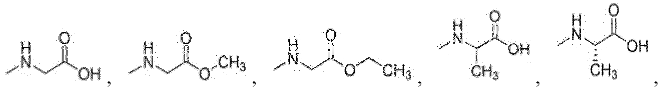 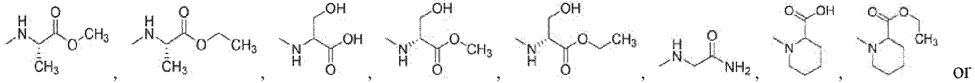 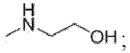 R³ and R⁴ preferably represent -CH₃ or Cl; R⁵ is preferably Cl; and R⁶ preferably represents -H or 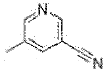 (5-cyanopyridin-3-yl).

In the compounds of the invention, X and Y represent O; *m*=1*, p*=1; A represents 1,4-substituted 1,2,3-triazole ring; L represents -CH₂O-, which has the following general formula (II): 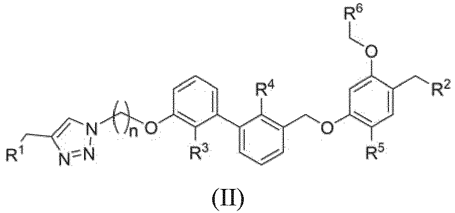 where *n*=2 or 3;
R¹ and R² each represents: OH, 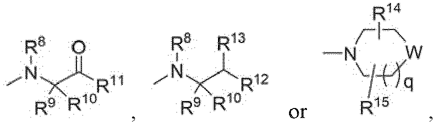 where R⁸ represents CH₃, CH₂CH₃, CH₂CH₂OH, formyl, acetyl, cyclopropyl, etc.; R⁹ and R¹⁰ each represents H, OH, COOH, CH₂COOH, CH₂NH₂, CH₂OH, CH₂CH₂OH, F, Cl, Br, CH₃, CH₂CH₃, cyclopropyl, etc.; R¹¹ represents OH, NH₂, NHCH₃, NHCH₂CH₃, CH₃, OCH₃, OCH₂CH₃, OC(CH₃)₃, OCH(CH₃)₂, 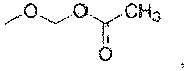 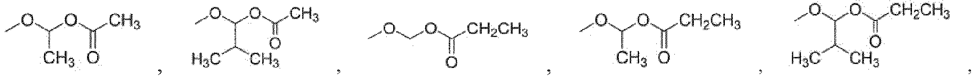 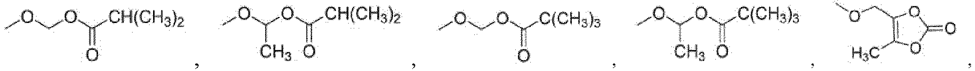 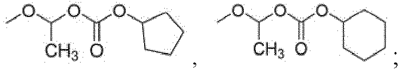 R¹² represents CONH₂, NHCOCH₃, OH, CH₂OH, CH₂CH₂OH, COOH, 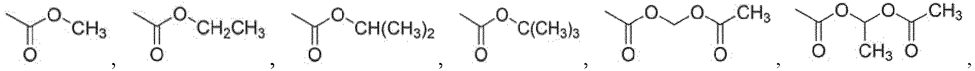 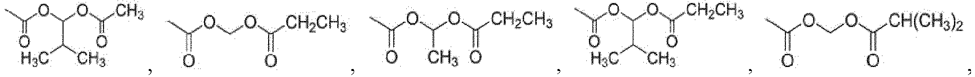 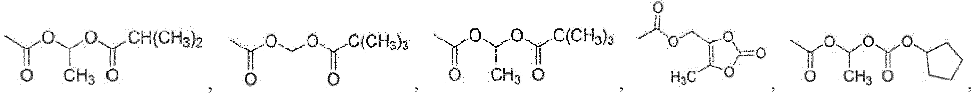 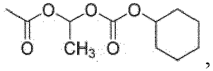 etc.; R¹³ represents H, CH₃, CH₂CH₃, CH₂OH, and/or CH₂CH₂OH, etc.; R¹⁴ and R¹⁵ each represents H, COOH, NH₂, F, Cl, Br, CH₃, CH₂CH₃, CH₂OH, CH₂CH₂OH, CONH₂, and/or cyclopropyl, 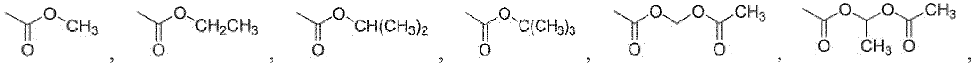 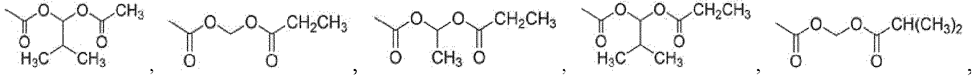 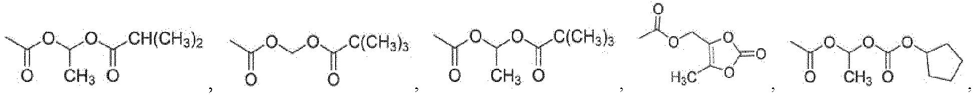 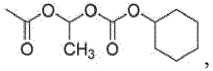 etc.; W represents CH₂, O, NH, N-CH₃, N-CH₂CH₃, N-CH₂CH₂OH, N-COCH₃, etc.; and q represents 0 or 1.
R³ and R⁴ each represent F, Cl, Br, CN or CH₃; R⁵ represents H, F, Cl, Br, CH₃ or OCH₃; and R6 represents H or

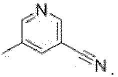

Further, each of R¹ and R² is preferably:

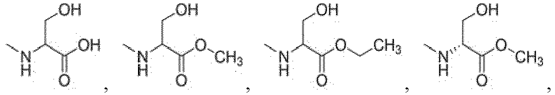

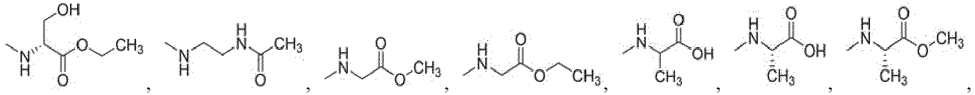

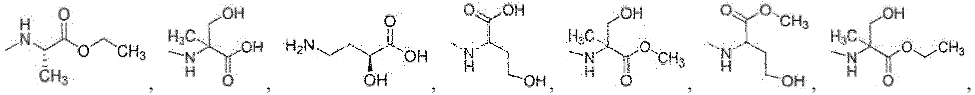

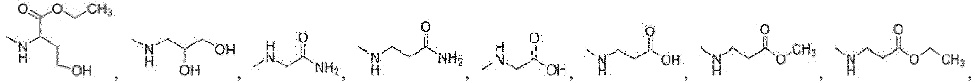

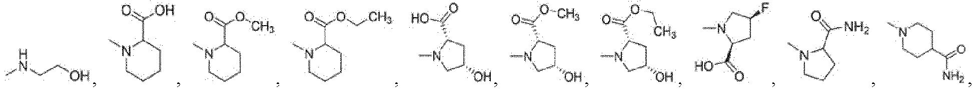

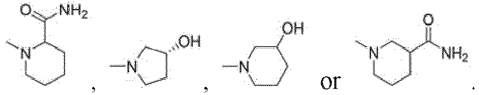

Further, each of R¹ and R² is preferably: 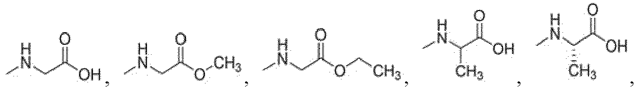 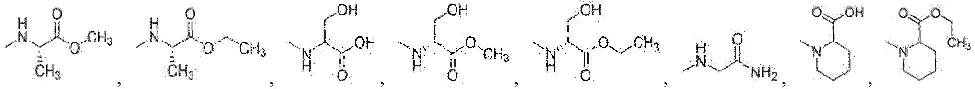 or 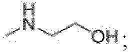 R³ and R⁴ are preferably CH₃, R⁵ is preferably Cl, and R⁶ is preferably H or

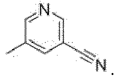

The biphenyl derivative represented by general formula (I) of the invention is preferably II-1, II-2, II-3, ... or 11-21 (21 compounds in all).

According to the above compounds or pharmaceutically acceptable salts thereof, the pharmaceutically acceptable salts are acid addition salts formed by the compound of general formula (I) and the following acids: hydrogen chloride, hydrogen bromide, sulfuric acid, carbonic acid, oxalic acid, citric acid, succinic acid, tartaric acid, phosphoric acid, lactic acid, pyruvic acid, acetic acid, maleic acid, methanesulfonic acid, benzenesulfonic acid, p-toluenesulfonic acid, or ferulic acid.

Compound (I) of the invention can be prepared by the following methods:
When X and Y represent O; *m*=1*, n*=2 or 3; *p*=1; A represents a 1,4-substituted 1,2,3-triazole ring; R⁵ represents Cl; L represents -CH₂O-; and the preparation method is as follows:
Preparation of intermediate IX: 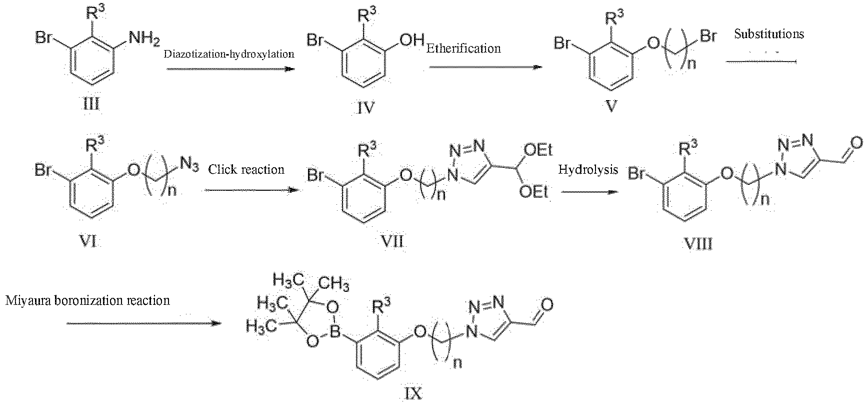 where a definition of R³ is the same as above.

Compound III is first formed salt with sulfuric acid; it then reacts with sodium nitrite to obtain diazonium salt, which was finally hydrolyzed to obtain compound IV. The reaction temperature is selected from 0 to 110°C, and preferably 25 to 100°C.

Compound V is prepared by reacting compound IV with 1,3-dibromopropane or 1,4-dibromobutane, and the base used is selected from sodium hydroxide, potassium hydroxide, potassium carbonate, sodium carbonate, cesium carbonate, sodium bicarbonate or potassium bicarbonate, preferably potassium carbonate; the solvent used is selected from acetone, acetonitrile, dioxane, *N*,*N*-dimethylformamide (DMF), dimethyl sulfoxide (DMSO) or a mixture of any two, and preferably acetone.

For the preparation of compound VI from compound V and azidotrimethylsilane, the solvent used is selected from acetone, acetonitrile, dioxane, *N*,*N*-dimethylformamide, dimethyl sulfoxide or a mixture of any two, and the solvent is preferably *N*,*N*-dimethylformamide; the reaction temperature is selected from 50 to 120°C, and preferably 60 to 100°C.

Compound VII is prepared by the Click reaction of compound VI and 3,3-diethoxy-1-propyne; the catalyst used is copper sulfate, copper chloride, copper bromide, cuprous chloride, cuprous bromide, iodine cuprous chloride or copper powder, preferably cuprous iodide. The solvents used are tetrahydrofuran, 1,4-dioxane, acetonitrile, ethanol, methanol, ethylene glycol dimethyl ether, dichloromethane, *N*,*N*-dimethylformamide, *N*,*N*-dimethylethyl ether amide (DMAc) or a mixed solvent of the above solvents and water, preferably acetonitrile and water. The reaction temperature is selected from 0 to 80°C, and preferably 20 to 50°C.

Compound VIII is prepared from compound VII under the action of acid, the acid used is selected from hydrogen chloride, hydrogen bromide, sulfuric acid, phosphoric acid, trifluoroacetic acid, methanesulfonic acid, benzenesulfonic acid, p-toluenesulfonic acid, preferably trifluoroacetic acid; and the solvent used is selected from ethyl acetate, tetrahydrofuran, dichloromethane, acetone, acetonitrile, dioxane, *N*,*N*-dimethylformamide, dimethyl sulfoxide or a mixed solvent of any two, preferably dichloromethane. The reaction temperature is selected from 0 to 80°C, and preferably 25 to 40°C.

Compound IX is prepared from compound VIII and bis(pinacolato)diboron, and the solvent used is selected from toluene, *N*,*N*-dimethylformamide, *N*,*N*-dimethylacetamide, ethylene glycol dimethyl ether, 1,4-dioxane, tetrahydrofuran, ethanol, acetonitrile, acetone, water or any mixed solvent composed of the two, preferably 1,4-dioxane; the base used is selected from sodium ethylate, sodium acetate, potassium acetate, potassium phosphate, potassium bicarbonate, potassium carbonate or triethylamine, preferably potassium acetate; the catalyst used is selected from tetrakis(triphenylphosphine)palladium (Pd(PPh₃)₄), [1,1'-bis(diphenylphosphino) ferrocene] palladium dichloride (Pd(dppf)Cl₂), bis(triphenylphosphine) palladium dichloride (Pd(PPh₃)₂Cl₂), palladium acetate (Pd(OAc)₂) or (1,1' - Bis(diphenylphosphino)ferrocene)nickel dichloride (NiCl₂(dppf)), preferably Pd(dppf)Cl₂; the reaction temperature is selected from 50 to 120°C, and preferably 60°C to 100°C.

Preparation of compound II: 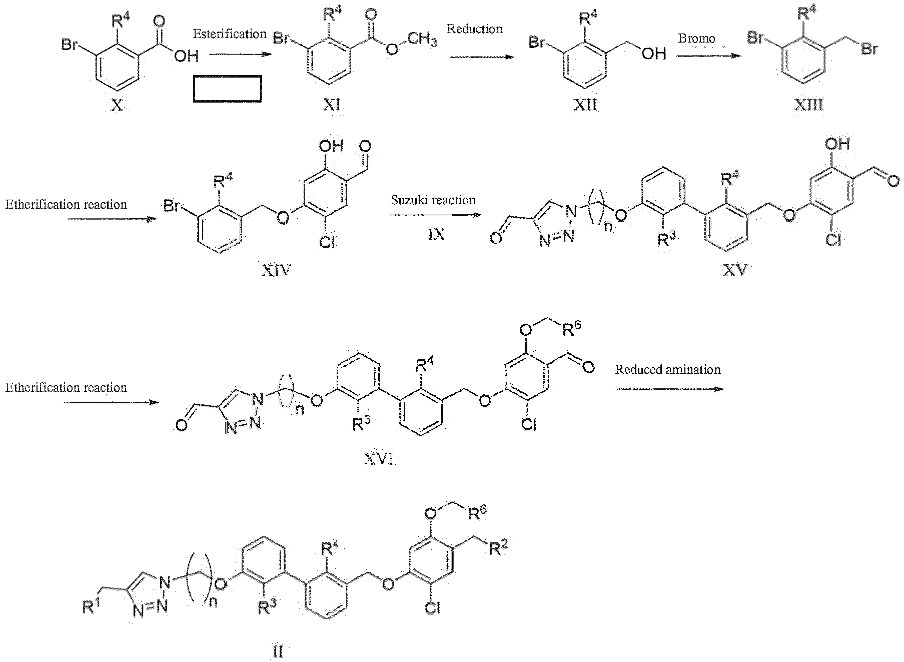 where definitions of R¹, R², R³, R⁴ and R⁶ are the same as above.

Compound X is reacted with SOCl₂ in methanol solution to prepare XI.

Compound XII is prepared from compound XI through reduction; the solvent used is selected from dichloromethane, tetrahydrofuran, methanol, ethanol, *N*,*N*-dimethylacetamide or dioxane, and preferably methanol; the selected reducing agent is selected from lithium aluminum tetrahydride, sodium borohydride or potassium borohydride, and preferably lithium aluminum tetrahydride. The reaction temperature is selected from -5 to 50°C, and preferably 0 to 25°C.

Compound XIII is prepared from compound XII by a bromination reaction; the brominated reagent used is selected from N-bromosuccinimide (NBS), phosphorus tribromide, carbon tetrabromide or hydrobromic acid, and preferably phosphorus tribromide. The solvent used is selected from dichloromethane, chloroform, carbon tetrachloride, tetrahydrofuran, acetonitrile or a mixed solvent composed of the above ones, preferably dichloromethane. The reaction temperature is selected from -5 to 50°C, and preferably 0 to 25°C.

Compound XIV is prepared from compound XIII and 5-chloro-2,4-dihydroxybenzaldehyde through a Williamson etherification reaction under alkaline conditions, and the solvent used is selected from acetone, *N*,*N-*dimethylformamide, dimethyl sulfoxide, acetonitrile, tetrahydrofuran or a mixed solvent composed of the above ones, and preferably acetonitrile; the base used is selected from sodium hydride, potassium tert-butoxide, sodium ethoxide, sodium methoxide, potassium carbonate, sodium carbonate, cesium carbonate, sodium hydroxide, potassium hydroxide , lithium hydroxide, sodium bicarbonate or potassium bicarbonate, preferably sodium bicarbonate. The reaction temperature is selected from 0°C to 100°C, and preferably 25-80°C.

Compound XV is prepared by the Suzuki reaction of compounds XIV and IX, and the solvent used is selected from toluene, *N*,*N*-dimethylformamide, *N*,*N*-dimethylacetamide, ethylene glycol dimethyl ether, 1,4-dioxane, tetrahydrofuran, methanol, ethanol, acetonitrile, acetone, water or a mixed solvent composed of any two solvents, preferably a mixed solvent of 1,4-dioxane and water; the base used is selected from sodium ethoxide and sodium acetate, potassium acetate, potassium phosphate, potassium bicarbonate, potassium carbonate or triethylamine, and preferably potassium carbonate; the catalyst used is selected from Pd(PPh₃)₄, Pd(dppf)Cl₂, Pd(PPh₃)₂Cl₂, Pd(OAc)₂ or NiCl₂(dppf), and preferably Pd(PPh₃)₄. The reaction temperature is selected from 50 to 120°C, and preferably 60°C to 100°C.

Compound XVI is prepared by the Williamson reaction of compound XV with iodomethane or 5-(chloromethyl)nicotinonitrile under alkaline conditions, and the solvent used is selected from acetone, *N*,*N*-dimethylformamide, acetonitrile, tetrahydrofuran or any mixed solvent of any two as above, and preferably acetonitrile; the base used is selected from sodium hydride, sodium methoxide, sodium ethoxide, potassium carbonate, sodium carbonate, cesium carbonate, sodium hydroxide, potassium hydroxide, sodium bicarbonate, potassium bicarbonate or hydroxide lithium, and preferably potassium carbonate. The reaction temperature is selected from 0 to 100°C, and preferably 25 to 80°C.

The target compound II is prepared by the reductive amination reaction of compound XVI and amine compounds, and the solvent used is selected from toluene, *N*,*N*-dimethylformamide, dichloromethane, dichloroethane, chloroform, carbon tetrachloride, methanol, 1,4-dioxane, tetrahydrofuran, ethanol, acetonitrile, acetone or a mixture composed of the above two solvents, and preferably a mixed solvent composed of dichloromethane and methanol; the reducing agent used is selected from triacetoxyboron sodium hydride, sodium cyanoborohydride, sodium borohydride, potassium borohydride or hydrosulfite, preferably sodium triacetoxyborohydride. The reaction temperature is selected from 0°C to 80°C, and preferably 0 to 50°C.

The invention also provides a pharmaceutical composition, which contains the above-mentioned compound of general formula (I) (including chiral isomers) or its pharmaceutically acceptable salt and a pharmaceutically acceptable carrier. The compound can be made into common pharmaceutical preparations or formulations by adding pharmaceutically acceptable carriers, such as tablets, capsules, syrups, suspensions, injections, and generally used pharmaceutical adjuvants, i.e. spices, sweeteners, liquid or solid fillers or diluents can be added.

The pharmaceutical composition of the invention can be administered by methods known to those skilled in the art, such as oral, rectal, sublingual, pulmonary, transdermal, iontophoresis, vaginal and intranasal administration. The pharmaceutical composition of the invention is preferably administered parenterally such as subcutaneous, intramuscular or intravenous injection. The dosage varies according to the form of the preparation, the expected duration of action, and the conditions of the subject to be treated. The therapeutically effective dose required for actual treatment can be determined by a doctor according to the actual situation (such as the patient's conditions, body weight, etc.). For an average adult, the dose of the pharmaceutical composition of the invention, based on the compound of general formula (I), may be 1 ng -10 g per kg body weight of an adult, preferably 10 µg - 100 mg per kg body weight.

The applications of the compounds of general formula (I) or their hydrate, solvate, crystal or pharmaceutically acceptable salts in the preparation of PD-1/PD-L1 protein-protein interaction inhibitor drugs are also protected within the scope of the current invention. Correspondingly, the invention also provides a treatment method, which covers administering a therapeutically effective dose of the compound of general formula (I) of the invention or its hydrates, solvates, crystals or pharmaceutically acceptable salts thereof to a subject in need of inhibition of the PD-1/PD-L1 interaction.

The invention also provides the use of the compounds of general formula (I) or their hydrates, solvates, crystals or pharmaceutically acceptable salts in the preparation of antitumor drugs. Correspondingly, the invention also provides a treatment method, which covers administering a therapeutically effective dose of the compound of general formula (I) of the invention or its hydrates, solvates, crystals or pharmaceutically acceptable salt(s) to a subject suffering from tumor.

Preferably, the PD-1/PD-L1 inhibitor of the invention can be used to prepare drugs for treating cancers such as non-small cell lung cancer, colon cancer or melanoma.

Compared with the prior art, the invention has the following significant beneficial effects:
(1) The novel biphenyl derivatives of the invention can significantly inhibit the interaction of PD-1/PD-L1, and its activity is significantly better than that of the known BMS-202. It is especially important that the biphenyl compounds of the invention can significantly block the inhibitory effect of PD-L1 on CD3⁺T cells, and has the effect of promoting T cells to secrete the immune factor INF-₇. In the co-incubation experiment of tumor cells and T cells, the promotion effect of the biphenyl compounds of the invention on the expression of INF-γ was significantly higher than that of the positive drug BMS-202, and was only slightly lower than that of pembrolizumab (5 µg/mL), and thus the compounds have an effect of enhancing the anti-tumor effect of T cells. Therefore, the biphenyl derivatives of the invention can be used as immune checkpoint PD-1/PD-L1 inhibitors for the preparation of drugs for tumor immunotherapy. Pharmacological experiments showed that in the homogeneous time-resolved fluorescence (HTRF) test, the biphenyl derivatives of the invention had a good inhibitory effect on the interaction of PD-1/PD-L1. In the surface plasmon resonance tests, the biphenyl derivatives of the invention had a good affinity to human PD-L1. The biphenyl derivatives of the invention can not only well inhibit the combination of PD-1/PD-L1, but also promote the recovery of T cell viability and the secretion of immune factor INF-γ, so it can be used for immunotherapy of tumors. The preferred 1,2,3-triazole heterocyclic derivatives of the invention are particularly excellent in activity, and have great practical significance and potential application prospects for the development of biphenyl PD-1/PD-L1 inhibitors.
(2) The synthesis route of the biphenyl derivatives of the invention is ingeniously designed, simple and easy to implement, the raw materials are inexpensive and readily available, the synthesis process is safe and environmentally friendly, and it is easy for large-scale production.
(3) It has wide applicability, and its drug as an active ingredient can be used to treat various cancers or tumors related to the immune checkpoint PD-1/PD-L1.
(4) The biphenyl derivatives of the invention have high pharmaceutical safety.

### Description of drawings

Figure 1 shows effects of compounds on expression of INF-γ in a co-culture experiment;
Figure 2 shows acute toxicity test results of compounds of the invention;
Figure 3 shows anti-tumor effects of compounds II-1 and II-18 on humanized MC38-hPD-L1 cell xenograft tumor model C57BL/6 mice (an effect of tumor volume);
Figure 4 shows anti-tumor effects of compounds II-1 and II-18 on humanized MC38-hPD-L1 cell xenograft tumor model C57BL/6 mice (an effect of tumor quality);
Figure 5 shows promoting effects of compounds 11-1 (25 mg/kg, ip, bid) and II-18 (25 mg/kg, po, bid) on CD3+CD4+T cell clustering in mice in vivo experiments; and
Figure 6 shows promoting effect of compounds II-1 (25 mg/kg, ip, bid) and 11-18 (25 mg/kg, po, bid) on CD3+CD8+T cell clustering in mice in vivo experiments.

### Specific Implementations

The invention will be described in detail below in conjunction with specific examples.

### Example 1

Synthesis of (4-((3'-(3-(4-(((1-carboxy-2-hydroxyethyl)amino)methyl)-1*H*-1,2,3-triazole-1-)propane oxy)-2,2'-dimethyl-[1,1'-biphenyl]-3-)methoxy)-5-chloro-2-((5-cyanopyridin-3-)methoxy)benzyl)serine (II-1: R¹ and R² are 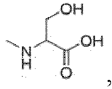 R³ and R⁴ are -CH₃, R⁵ is Cl, R⁶ is 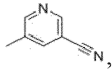 *n=3)*

### Synthesis of 3-bromo-2-methylphenol (IV-1)

3-bromo-2-methylaniline (5.00 g, 26.9 mmol) was added into a three-necked flask, and a sulfuric acid solution (65 mL, 1 mmol/L) was slowly added dropwise during stirring; a large amount of insoluble white solids precipitated. After drop addition, an internal temperature of a reaction solution was lowered to 0-5°C, a 30% sodium nitrite solution (2.23 g, 32.3 mmol) was slowly added dropwise; yellow insoluble matter was formed; after adding is completed, the temperature was lowered to 0-5°C and stirring was conducted for 30 min. Toluene (30 mL) was added, and the temperature was raised to 100 °C for 1 h. After the reaction solution became clear and transparent, the reaction was stopped, cooling was conducted to a room temperature, extraction was conducted with ethyl acetate (50 mL × 3), organic phases were combined, washing was conducted with water (30 mL × 2) and a saturated sodium chloride solution (20 mL), separately, and washing was conducted with anhydrous sulfuric acid and then drying was conducted over anhydrous sodium sulfate, and then suction filtration was conducted, solvent was removed under reduced pressure, and finally purification was conducted through column chromatography (petroleum ether:ethyl acetate=100:1); 4.83 g of white needle crystals were obtained with a yield of 96.1%, m.p. at 95.0-98.0°C.

¹H NMR (300 MHz, Chloroform-*d*) δ 7.19 (dd, *J* = 8.0, 1.2 Hz, 1H, ArH), 6.97 (t, *J=* 8.0 Hz, 1H, ArH), 6.76 (d, *J* = 7.9 Hz, 1H, ArH), 4.82 (s, 1H, OH), 2.38 (s, 3H, CH₃).

### Synthesis of 1-(1-bromo-3-(3-bromopropoxy)-2-methylbenzene (V-1)

3-bromo-2-methylphenol (5.00 g, 26.7 mmol), 1,3 dibromopropane (8.13 mL, 80.1 mmol), potassium carbonate (9.23 g, 66.8 mmol) were dissolved in acetone (50 mL), then heating was conducted to reflux; after 4 h of reaction, the reaction was completed (monitored by TLC(petroleum ether:ethyl acetate=15:1)). Heating was stopped and then cooling was conducted to a room temperature. The reaction solution was poured into ice water (100 mL), extracted with ethyl acetate (50 mL × 3), the combined organic phases were washed with water (30 mL × 2) and saturated sodium chloride solution (20 mL × 2), separately; then drying was conducted over anhydrous sodium sulfate, suction filtration was conducted, and the solvent was removed under reduced pressure to obtain a crude product, which was purified through column chromatography (petroleum ether 100%) to obtain 6.98 g of a colorless transparent liquid with a yield of 84.8%.

¹H NMR (300 MHz, Chloroform-*d*) δ 7.16 (dd, *J* = 8.1, 1.2 Hz, 1H, ArH), 7.00 (td, *J=* 8.1, 0.6 Hz, 1H, ArH), 6.79 (dd, *J* = 8.2, 1.2 Hz, 1H, ArH), 4.10 (t, *J* = 5.7 Hz, 2H, OCH₂), 3.62 (t, *J* = 6.4 Hz, 2H, BrCH₂), 2.39-2.32 (m, 2H, CH₂CH₂CH₂), 2.31 (s, 3H, CH₃).

### Synthesis of 1-(3-azidopropoxy)-3-bromo-2-methylbenzene (VI-1)

Compound V-1 (6.00 g, 19.5 mmol), azidotrimethylsilane (3.08 mL, 23.4 mmol), CsF (4.44 g, 29.3 mmol) and DMF (30 mL) were sequentially added to an eggplant-shaped flask which was then heated to 50 °C. After 4 h of reaction, the reaction of raw materials was completed (monitored by TLC(petroleum ether:ethyl acetate = 30:1)); heating was stopped, and the system was cooled to a room temperature. The reaction solution was slowly poured into ice water (60 mL), extracted with ethyl acetate (40 mL × 3), the combined organic phases were washed with water (40 mL × 2) and saturated sodium chloride solution (30 mL × 2), separately; then drying was conducted over anhydrous sodium sulfate, suction filtration was conducted, and the solvent was removed under reduced pressure to obtain a crude product, which was purified through column chromatography to obtain 4.91 g of a colorless transparent liquid with a yield of 93.4%.

¹H NMR (300 MHz, Chloroform-*d*) δ 7.22 (dd, *J* = 8.1, 1.2 Hz, 1H, ArH),7.11 (td, *J* = 8.1, 3.6 Hz, 1H,ArH), 6.83 (dd, *J* = 8.2, 1.1 Hz, 1H, ArH), 4.10 (t, *J* = 5.9 Hz, 2H, OCH₂), 3.60 (t, *J* = 6.7 Hz, 2H, CH₂N₃), 2.34 (d, *J*= 5.7 Hz, 3H, CH₃), 2.21 - 2.07 (m, 2H, CH₂CH₂CH₂).

### Synthesis of 1-(3-(3-bromo-2-methylphenoxy)propyl)-4-(diethoxymethyl)-1H-1,2,3-triazole (VII-1)

Compound VI-1 (4.50 g, 16.7 mmol), 3,3-diethoxy-1-propyne (2.88 mL, 20.1 mmol), cuprous iodide (0.16 g, 0.84 mmol) and acetonitrile (30 mL) were sequentially added into the eggplant-shaped flask, and the temperature was raised to 30°C for reaction. The reaction of the raw materials was completed (monitored by TLC (petroleum ether:ethyl acetate = 8:1)); heating was stopped, and the system was cooled to a room temperature. The insoluble matter was removed by suction filtration; the filtrate was slowly poured into water (50 mL), extracted with ethyl acetate (30 mL × 3), the organic phases were combined, and washed with water (30 mL × 2) and saturated sodium chloride solution (30 mL × 2); then drying was conducted over anhydrous sodium sulfate, suction filtration was conducted, and the solvent was removed under reduced pressure to obtain a crude product, which was purified by column chromatography to obtain 4.89 g of a colorless transparent liquid with a yield of 73.8%, which was directly used in the next step of the reaction.

### Synthesis of 1-(3-(3-bromo-2-methylphenoxy)propyl)-1H-1,2,3-triazole-4-carbaldehyde (VIII-1)

Compound VII-1 (4.50 g, 11.3 mmol) and dichloromethane (30 mL) were sequentially added to an eggplant-shaped flask, and trifluoroacetic acid (3.54 mL, 47.6 mmol) was slowly added dropwise. After stirring at a room temperature for 1 h, the reaction of raw materials was completed (monitored by TLC (petroleum ether:ethyl acetate=4:1)), then the pH was adjusted to 7 by using saturated sodium bicarbonate. Extraction was conducted with dichloromethane (30 mL × 2), the organic phases were combined, washing was conducted with water (30 mL × 2) and saturated sodium chloride solution (30 mL × 2), separately; then drying was conducted over anhydrous sodium sulfate, suction filtration was conducted, and the solvent was removed under reduced pressure to obtain crude product, which was purified by column chromatography (petroleum ether:ethyl acetate=6:1) to obtain 3.25 g of white solid powder with a yield of 89.0%. m.p. at 94.0-96.0 °C.

¹H NMR (300 MHz, Chloroform-*d*) δ 10.15 (s, 1H, CHO), 8.12 (s, 1H, ArH), 7.19 (d, *J* = 8.0 Hz, 1H, ArH), 7.00 (t, *J* = 8.1 Hz, 1H, ArH), 6.72 (d, *J* = 8.2 Hz, 1H, ArH), 4.69 (t, *J* = 7.0 Hz, 2H, NCH₂), 4.00 (t, *J* = 5.7 Hz, 2H, OCH₂), 2.50 (p, *J* = 6.3 Hz, 2H, CH₂CH₂CH₂), 2.33 (s, 3H, CH₃).

### Synthesis of 1-(3-(2-methyl-3-(4,4,5,5-tetramethyl-1,3,2-dioxaborolan-2-)phenoxy)propyl)-1H-1,2,3-triazole-4-carbaldehyde (IX-1)

Compound VIII-1 (3.00 g, 9.25 mmol), bis(pinacolato)diboron (2.83 g, 11.1 mmol), potassium acetate (2.28 g, 23.2 mmol) were dissolved in 1,4-dioxane (15 mL) , Pd(dppf)Cl PPh₃₂ (0.34 g, 0.47 mmol) was added under the protection of N₂, and the temperature was raised to 80°C for 12 h. The reaction of raw materials was completed (monitored by TLC (petroleum ether:ethyl acetate=4:1)); heating was stopped, and the system was cooled to a room temperature. The reaction solution was slowly poured into ice water (30 mL), and extraction was conducted with ethyl acetate (20 mL × 3); the organic phases were combined, and washing was conducted with water (20 mL × 2) and saturated sodium chloride solution (20 mL × 2), separately; then drying was conducted over anhydrous sodium sulfate, and suction filtration was conducted. The solution was then removed from the solvent under reduced pressure to obtain a crude product, which was purified by column chromatography to obtain 2.71g of a white solid powder with a yield of 78.9%. m.p. at 137.0-139.0 °C

¹H NMR (300 MHz, Chloroform-*d*) *δ* 10.02 (s, 1H, CHO), 8.94 (s, 1H, ArH), 7.20 (dd, *J* = 7.4, 1.2 Hz, 1H, ArH), 7.12 (t, *J*= 7.5 Hz, 1H, ArH), 6.99 (dd, *J*= 8.2, 1.6 Hz, 1H, ArH), 4.66 (t, *J*= 6.9 Hz, 2H, NCH₂), 3.97 (t, *J* = 5.9 Hz, 2H, OCH₂), 2.38 (p, *J* = 6.5 Hz, 2H, CH₂CH₂CH₂), 2.30 (s, 3H, ArCH₃), 1.30 (s, 12H, CH₃).

### Synthesis of 3-bromo-2-methylbenzoate methyl ester (XI-1)

3-Bromo-2-methylbenzoic acid (X-1) (10.0 g, 46.7 mmol) was dissolved in methanol (50 mL), and cooled to 0 °C. Thionyl chloride (4.07 mL, 56.1 mmol) was slowly added dropwise, and after this step was completed, the temperature was raised to 65 °C and the system was refluxed for 3 h. Next, after the reaction of raw materials was completed (monitored by TLC(petroleum ether:ethyl acetate=8:1)), heating was stopped and the solution was cooled to room temperature. The solvent was distilled off under reduced pressure to obtain 10.65 g of white solid powder with a yield of 99.9%. m.p. at 31.0-33.0°C. (Values in the literature: 30-31°C (1) Zhang, Peng; Journal of Medicinal Chemistry 2010, V53(16), P6112-6121 CAPLUS) .

¹H NMR (300 MHz, Chloroform-*d*) δ 7.72 (d, *J* = 8.5 Hz, 1H, ArH), 7.68 (d, *J* = 8.6 Hz, 1H, ArH), 7.09 (t, *J* = 7.8 Hz, 1H, ArH), 3.90 (s, 3H, OCH₃), 2.63 (s, 3H, CH₃).

### Synthesis of 3-bromo-2-methylbenzyl alcohol (XII-1)

Compound XI-1 (10.50 g, 46.1 mmol) was dissolved in anhydrous tetrahydrofuran (50 mL), under the protection of nitrogen, the temperature was lowered to 0°C, and LiAlH₄ (3.10 g, 55.3 mmol) was slowly added in batches. After the addition was completed, the ice bath was removed, and the system was stirred at r.t. for 30 min. The reaction of raw materials was completed (monitored by TLC(petroleum ether:ethyl acetate=8:1)), and saturated NH₄Cl solution was slowly added dropwise until no bubbles were formed, the solution was diluted with ethyl acetate (100 mL), insoluble matter was removed by suction filtration, and the organic phase was washed with water (50 mL × 2) and then saturated sodium chloride solution (50 mL × 2); next, then drying was conducted over anhydrous sodium sulfate, suction filtration was conducted, and heating was stopped, the solvent was cooled under reduced pressure to obtain 9.21 g of white solid powder with a yield of 99.9%. m.p. at 103.0-104.0°C.

¹H NMR (300 MHz, DMSO-*d*₆) δ 7.49 (d, *J*= 8.0 Hz, 1H, ArH), 7.39 (d, *J* = 7.7 Hz, 1H, ArH), 7.12 (t, *J=* 7.7 Hz, 1H, ArH), 5.26 (br, 1H, CH₂OH), 4.52 (s, 2H, CH₂), 2.30 (s, 3H, CH₃).

### Synthesis of 1-bromo-3-(bromomethyl)-2-methylbenzene (XIII-1)

Compound XII-1 (2.58 g, 12.8 mmol) was dissolved in dichloromethane (20 mL), and cooled to 0°C. Phosphorus tribromide (1.82 mL, 19.2 mmol) was slowly added and reacted overnight at a room temperature. Next, the reaction was completed (monitored by TLC (petroleum ether:ethyl acetate=15:1-8:1)), and a small polar point was formed. Saturated sodium bicarbonate was used to adjust the pH to alkaline, the solution was extracted with dichloromethane (20 mL × 3), washed with saturated sodium chloride solution (30 mL × 3), washed with water (30 mL × 3), and dried over anhydrous sodium sulfate; the solvent was distilled off under reduced pressure to obtain 3.30 g of a colorless transparent oily liquid with a yield of 97.4%. m.p. at 30.0-31.5°C.

¹H NMR (300 MHz, Chloroform-*d*) δ 7.52 (dd, *J* = 8.0, 1.5 Hz, 1H, ArH), 7.27 (s, 1/2H, ArH), 7.24 (s, 1/2H, ArH), 7.02 (t, *J*= 7.8 Hz, 1H, ArH), 4.52 (s, 2H, CH₂), 2.48 (s, 3H, CH₃).

### Synthesis of 4-((3-bromo-2-methylbenzyl)oxy)-5-chloro-2-hydroxybenzaldehyde (XIV-1)

Compound XIII-1 (5.00 g, 18.9 mmol), 5-chloro-2,4-dihydroxybenzaldehyde (3.27 g, 18.9 mmol), sodium bicarbonate (3.97 g, 47.3 mmol) were dissolved in acetonitrile (50 mL); the system was heated up to 80°C, after reacting for 12 h, TLC (petroleum ether:ethyl acetate=8:1-4:1) monitoring was done to generate new points, then the reaction was stopped. The reaction solution was poured into water (150 mL), suction filtration was conducted to obtain a white powder solid, and drying was conducted in a vacuum. The crude product was subjected to column chromatography to obtain 1.87 g of white solid powder with a yield of 27.5%. m.p. at 178.0-181.0°C.

¹H NMR (300 MHz, Chloroform-*d*) δ 11.20 (s, 1H, ArOH), 10.04 (s, 1H, CHO), 7.57 (d, *J* = 8.1 Hz, 1H, ArH), 7.41 (d, *J* = 7.5 Hz, 1H, ArH), 7.09 (t, *J* = 7.8 Hz, 1H, ArH), 6.88 (s, 1H, ArH), 5.10 (s, 2H, CH₂), 2.45 (s, 3H, CH₃).

### Synthesis of 1-(3-((3'-((2-chloro-4-formyl-5-hydroxyphenoxy)methyl)-2,2'-dimethyl-[1,1'-biphenyl]-3-yl)oxy)propyl)-1H -1,2,3-triazole-4-carbaldehyde (XV-1)

Compound XIV-1 (5.20 g, 14.7 mmol), compound IX-1 (4.97 g, 13.4 mmol) and 1, 4-dioxane (60 mL) were sequentially added to an eggplant-shaped flask, and potassium carbonate (5.18 g, 37.5 mmol) was dissolved in water (6 mL) and added to the reaction solution; then, Pd(PPh₃)₄ (1.55 g, 1.34 mmol) was added under nitrogen protection, and the system was reacted at 80°C for 12 hours. A white insoluble matter was precipitated, and the reaction of raw materials was completed (monitored by TLC (petroleum ether:ethyl acetate = 4:1)); then heating was stopped and the system was cooled to a room temperature. The reaction solution was poured into water, extraction was conducted with ethyl acetate (50 mL × 3), the organic phases were combined, washing was conducted with water (50 mL × 2) and saturated sodium chloride solution (50 mL × 2), and drying was conducted over anhydrous sodium sulfate followed by suction filtration; next, the solvent was removed under reduced pressure to obtain a brown-black solid, which was purified by column chromatography (petroleum ether:ethyl acetate=2:1) to obtain 3.70 g of yellow solid powder with a yield of 48.6%. m.p. at 161.0-164.0°C.

¹H NMR (400 MHz, DMSO-*d*₆) δ 11.20 (d, *J* = 1.9 Hz, 1H, OH), 10.05 (s, 1H, CHO), 10.02 (s, 1H, CHO), 8.96 (s, 1H, ArH), 7.71 (s, 1H, ArH), 7.51 (dd, *J* = 7.8, 1.5 Hz, 1H, ArH), 7.30 (t, *J* = 7.6 Hz, 1H, ArH), 7.21 (t, *J*= 7.9 Hz, 1H, ArH), 7.09 (dd, *J*= 7.6, 1.6 Hz, 1H, ArH), 6.94 (dd, *J* = 8.4, 1.1 Hz, 1H, ArH), 6.89 (s, 1H, ArH), 6.69 (dd, *J* = 7.6, 1.1 Hz, 1H, ArH), 5.32 (s, 2H, ArOCH₂), 4.69 (t, *J* = 6.9 Hz, 2H, NCH₂CH₂), 4.16 - 3.95 (m, 2H, OCH₂CH₂), 2.41 (p, *J* = 6.8 Hz, 2H, CH₂CH₂CH₂), 2.01 (s, 3H, CH₃), 1.80 (s, 3H, CH₃).

### Synthesis of 5-((4-chloro-2-formyl-5-((3'-(3-(4-formyl-1H-1,2,3-triazol-1-)propoxy)-2,2'-Dimethyl-[1,1'-biphenyl]-3-)methoxy)phenoxy)methyl)nicotinonitrile (XVI-1)

Compound XV (2.88 g, 5.54 mmol), 5-(chloromethyl) nicotinonitrile (1.15 g, 6.10 mmol), cesium carbonate (5.40 g, 16.7 mmol), potassium iodide (0.05 g, 0.28 mmol) and *N,N*- dimethylformamide (15 mL) was sequentially added into an eggplant-shaped flask, and the temperature was raised to 80°C for 6 h of reaction. After the reaction of raw materials was completed (monitored by TLC), heating was stopped and the system cooled to a room temperature. The reaction solution was slowly poured into ice water (50 mL), and solids were precipitated by suction filtration; then, the filtrate was extracted with ethyl acetate (20 mL × 3), the organic phases were combined, and washing was conducted with water (20 mL × 2) and saturated sodium chloride solution (20 mL × 2), drying was conducted over anhydrous sodium sulfate, suction filtration was conducted, and the solvent was removed under reduced pressure to obtain a brownish black solid, which was purified by column chromatography (petroleum ether:ethyl acetate=15:1-8:1), 2.29 g of a yellow solid powder were obtained, and the yield was 65.0%. m.p. at 57.0-59.0°C.

¹H NMR (400 MHz, DMSO-*d*₆) δ 10.20 (s, 1H, CHO), 10.02 (s, 1H, CHO), 9.06 (s, 1H, ArH), 8.87 (d, *J* = 2.0 Hz, 1H, ArH), 8.51 (d, *J* = 2.4 Hz, 1H, ArH), 8.30 (t, *J* = 2.2 Hz, 1H, ArH), 7.73 (s, 1H, ArH), 7.53 (dd, *J* = 7.7, 1.4 Hz, 1H, ArH), 7.34 - 7.27 (m, 2H, ArH), 7.21 (t, *J* = 7.9 Hz, 1H, ArH), 7.10 (dd, *J* = 7.7, 1.4 Hz, 1H, ArH), 6.94 (d, *J* = 8.0 Hz, 1H, ArH), 6.70 (d, *J*= 7.4 Hz, 1H, ArH), 5.45 (s, 2H, ArOCH₂), 5.43 (d, *J* = 2.5 Hz, 2H, ArOCH₂), 4.69 (t, *J*= 6.9 Hz, 2H, NCH₂), 4.15 - 4.04 (m, 2H, OCH₂), 2.41 (p, *J* = 6.6 Hz, 2H, CH₂CH₂CH₂), 2.04 (s, 3H, ArCH₃), 1.82 (s, 3H, ArCH₃).

### Synthesis of (4-((3'-(3-(4-(((1-carboxy-2-hydroxyethyl)amino)methyl)-1H-1,2,3-triazol-1-yl)propane oxy)-2,2'-dimethyl-[1,1'-biphenyl]-3-yl)methoxy)-5-chloro-2-((5-cyanopyridin-3-)methoxy)benzyl)serine (II-1)

Compound XVI-1 (0.28 g, 0.44 mmol) and serine methyl ester hydrochloride (274 mg, 1.76 mmol) were dissolved in dichloromethane (10 mL), stirred at a room temperature for 30 min, and NaBH(OAc)₃ (1.49 g, 7.04 mmol) was slowly added; the system reacted at room temperature for 2 hours. Next, the reaction of raw materials was completed (monitored by TLC), and the reaction was stopped. The pH was adjusted to 7 with a saturated sodium bicarbonate solution, extraction was conducted with dichloromethane (10 mL × 2), the organic phases that were combined were washed with water (10 mL × 2) and saturated sodium chloride solution (10 mL × 2), separately; next, the solution was then dried over anhydrous sodium sulfate, suction filtration was conducted, and the solvent was removed under reduced pressure to obtain a brownish-black solid, which was purified by TLC (dichloromethane: Methanol=20:1), to obtain 120 mg of viscous colorless liquid. This liquid was dissolved in methanol (1 mL) and tetrahydrofuran (1 mL) and added to an eggplant-shaped flask after which, in turn, lithium hydroxide monohydrate (20 mg, 0.45 mmol) was added, and the system reacted at a room temperature for 3 h. Next, After the reaction of the raw materials was completed (monitored by TLC (dichloromethane: Methanol=10:1)), then the organic solvent was evaporated under reduced pressure, the pH of the residue adjusted to 6-7 with 0.5 M NaOH, and solid precipitated, suction filtration was conducted, and drying was conducted to obtain 70 mg of brown solid powder with a yield of 19.5%.

1H NMR (300 MHz, DMSO-*d*₆) δ 8.78 (s, 1H, ArH), 8.73 (s, 1H, ArH), 8.31 (s, 1H, ArH), 8.25 (s, 1H, ArH), 7.60 (s, 1H,ArH), 7.51 (d, *J* = 7.08 Hz, 1H, ArH), 7.31 - 7.26 (m, 1H, ArH), 7.25 - 7.20 (m, 2H, ArH), 7.10 (d, *J* = 7.4 Hz, 1H, ArH), 6.96 (d, *J* = 8.0 Hz, 1H, ArH), 6.72 (d, *J* = 7.2 Hz, 1H, ArH), 5.33 (s, 4H, ArOCH₂), 4.62 (t, *J* = 6.6 Hz, 2H, NCH₂), 4.19 - 4.17 (m, 2H, ArCH₂), 4.10 (s, 2H, ArCH₂), 4.06 (d, *J* = 6.4 Hz, 2H, OCH₂), 3.88 - 3.69 (m, 4H, 2CH₂OH), 3.64 - 3.41 (m, 2H, CH), 2.37 - 2.34 (m, 2H, CH₂CH₂CH₂), 2.06 (s, 3H, ArCH₃), 1.84 (s, 3H, ArCH₃).

HRMS (ESI): m/z [M+H]⁺. Calcd for C₄₁H₄₅ClN₇O₉: 814.2967, found: 814.2987.

### Example 2

Synthesis of 5-((4-chloro-2-(((2-hydroxyethyl)amino)methyl)-5-((3'-(3-(4-(((2-hydroxyethyl)amino)methyl Base)-1*H*-1,2,3-triazol-1-)propoxy)-2,2'-dimethyl-[1,1'-biphenyl]-3-)methoxy)phenoxy) methyl)nicotinonitrile (II-2: R¹ and R² are 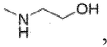 R³ and R⁴ are -CH₃, R⁵ is Cl, R⁶ is 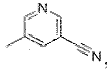 *, n*=3)

Compound XVI-1 (0.20 g, 0.31 mmol) and ethanolamine (57 µL, 0.95 mmol) were dissolved in a mixed solution of dichloromethane (4 mL) and methanol (2 mL); the solution was then stirred at a room temperature for 30 min, and NaBH(OAc)₃ (0.81 g, 3.80 mmol) was added slowly; the system was allowed to react at a room temperature for 2 h. Next, after the reaction of raw materials was completed (monitored by TLC), and the reaction was stopped. Next, the pH was adjusted to 7 with saturated sodium bicarbonate solution, extracted with dichloromethane (10 mL × 2), the organic phases were combined, and the solution was washed with water (10 mL × 2) and a saturated sodium chloride solution (10 mL × 2), separately; and then drying was conducted over anhydrous sodium sulfate, suction filtration was conducted, and the solvent was removed under reduced pressure to obtain a light yellow viscous liquid, which was purified by TLC (dichloromethane: Methanol=20:1-15:1); 32 mg of yellow solid powder was obtained with a yield of 12.9%, m.p. at 61.0-64.0°C.

¹H NMR (400 MHz, Chloroform-*d*) δ 8.68 (s, 1H, ArH), 8.61 (s, 1H, ArH), 7.96 (s, 1H, ArH), 7.54 (s, 1H, ArH), 7.47 (dd, *J* = 7.6, 1.4 Hz, 1H, ArH), 7.34 (s, 1H, ArH), 7.31 - 7.29 (m, 1H, ArH), 7.28-7.27 (m, 1H, ArH), 7.20 (t, *J*= 7.9 Hz, 1H, ArH), 7.15 (dd, *J*= 7.6, 1.5 Hz, 1H, ArH), 6.80 (dd, *J*= 11.5, 7.4 Hz, 1H, ArH), 6.63 (s, 1H, ArH), 5.17 (s, 2H, ArCH₂O), 5.07 (s, 2H, ArCH₂O), 4.63 (t, *J*= 6.9 Hz, 2H, NCH₂), 4.15 - 4.05 (m, 2H, OCH₂CH₂CH₂), 3.95 (s, 2H, ArCH₂NH), 3.81 (s, 2H, ArCH₂NH), 3.73 - 3.65 (m, 4H, CH₂OH), 2.88 - 2.83 (m, 4H, NHCH₂CH₂OH) 2.47 (p, *J=* 6.9 Hz, 2H, CH₂CH₂CH₂), 2.10 (s, 3H, ArCH₃), 1.94 (s, 3H, ArCH₃).

### Example 3

Synthesis of N (2-((4-((3'-(3-(4-(((2-acetylaminoethyl)amino) methyl)-1*H*-1,2,3-triazole-1-)propoxy)-2,2'-dimethyl-[1,1'-biphenyl]-3-)methoxy)-5-chloro-2-((5-cyanopyri dine-3-)methoxy) benzyl)amino)ethyl)acetamide (II-3: R¹ and R² are 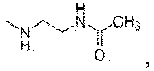 R³ and R⁴ are -CH₃, R⁵ is Cl, and R⁶ is 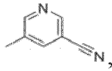 *n=3)*

Compound XVI-1 (0.20 g, 0.31 mmol) and N-acetylethylenediamine (0.10 g, 0.95 mmol) were dissolved in dichloromethane (5 mL) and added to the reaction flask successively, stirring was conducted at a room temperature for 30 min, and NaBH(OAc)₃ was added slowly (OAc)₃ (0.81 g, 3.80 mmol), and then reacted at a room temperature for 2 h. The reaction of raw materials was completed (monitored by TLC), and the reaction stopped. The pH was adjusted to 7 with saturated sodium bicarbonate solution, extracted with dichloromethane (10 mL × 2), the organic phases were combined, washed with water (10 mL × 2) and saturated sodium chloride (10 mL × 2), and then dried over anhydrous sodium sulfate, suction filtration was conducted, and the solvent was removed under reduced pressure to obtain a crude product, which was separated by column chromatography (dichloromethane: Methanol=50:1-20:1) to obtain a light yellow viscous liquid, which was purified by TLC (dichloromethane: Methanol=20:1-15:1) to obtain 61 mg of yellow solid powder with a yield of 24.0%. m.p. at 68.0-70.0 °C.

¹H NMR (400 MHz, Chloroform-*d*) δ 8.69 (s, 1H, ArH), 8.63 (s, 1H, ArH), 7.97 (s, 1H, ArH), 7.55 (s, 1H, ArH), 7.45 (d, *J* = 7.2 Hz, 1H, ArH), 7.37 (s, 1H, ArH), 7.29-7.21 (m, 1H, ArH), 7.20 (t, *J* = 7.9 Hz, 1H, ArH), 7.15 (d, *J* = 7.6 Hz, 1H, ArH), 6.80 (dd, *J* = 13.0, 7.9 Hz, 2H, ArH), 6.64 (s, 1H, ArH), 6.51 (s, 1H, CONH), 6.42 (s, 1H, CONH), 5.16 (s, 2H, ArCH₂O), 5.10 (s, 2H, ArCH₂O), 4.63 (t, *J*= 6.9 Hz, 2H, NCH₂), 4.10-4.01 (m, 2H, OCH₂), 3.93 (s, 2H, ArCH₂NH), 3.82 (s, 2H, ArCH₂NH), 3.43-3.35 (m, 4H, CONHCH₂), 3.07 (s, 2H, NH), 2.82 (t, *J* = 5.8 Hz, 4H, NHCH₂CH₂), 2.48-2.35 (m, 2H, CH₂CH₂CH₂), 2.10 (s, 3H, ArCH₃), 1.98 (s, 3H, ArCH₃), 1.97 (s, 3H, CH₃), 1.94 (s, 3H, CH₃).

### Example 4

Synthesis of 1-(4-((3'-(3-(4-((2-carbamoylpyrrolidin-1-)methyl)-1*H*-1,2,3-triazol-1-) Propoxy)-2,2'-dimethyl-[1,1'-biphenyl]-3-methoxy)-5-chloro-2-((5-cyanopyridine-3-)methoxy)benzyl) pyrrolidine-2-carboxamide (II-4: R¹ and R² are 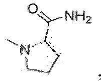 R³ and R⁴ are -CH₃, R⁵ is Cl, R⁶ is 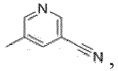 *n=3)*

Compound XVI-1 (0.20 g, 0.31 mmol) and L-prolineamide (0.11 g, 0.95 mmol) were dissolved in dichloromethane (5 mL), added to the reaction flask successively, and then stirred at a room temperature for 30 min. Next, NaBH(OAc)₃ (0.81 g, 3.80 mmol) was slowly added and allowed to react at a room temperature for 2 h. The presence of a large amount of raw materials was monitored by TLC (dichloromethane: Methanol=10:1), NaBH(OAc)₃ (0.40 g, 1.90 mmol) was added; the reaction continued for 4 h, and the reaction was stopped. The pH was adjusted to 7 with saturated sodium bicarbonate solution, the solution was extracted with dichloromethane (10 mL × 2), the organic phases were combined, washing was conducted with water (10 mL × 2) and saturated sodium chloride (10 mL × 2), and drying was conducted over anhydrous sodium sulfate, suction filtration was conducted, and the solvent was removed under reduced pressure to obtain a crude product, which was separated by column chromatography (dichloromethane:methanol=50:1-20:1) to obtain a light yellow viscous liquid, which was then purified by TLC (dichloromethane:methanol=15:1-10:1), 53 mg of yellow solid powder was obtained with a yield of 20.3%. m.p. at 98.0-101.0°C.

¹H NMR (400 MHz, Chloroform-*d*) δ 8.68 (s, 1H, ArH), 8.59 (s, 1H, ArH), 7.94 (s, 1H, ArH), 7.47-7.45 (m, 2H, ArH), 7.31 (s, 1H, CONH₂), 7.26 (d, *J* = 7.5 Hz, 2H, ArH), 7.19 (t, *J* = 7.9 Hz, 1H, ArH), 7.14 (d, *J* = 7.6 Hz, 1H, ArH), 6.82 (d, *J* = 8.1 Hz, 1H, ArH), 6.78 (d, *J* = 7.6 Hz, 1H, ArH), 6.65-6.60 (m, 1H, ArH), 5.76 (d, *J* = 4.0 Hz, 1H, CONH₂), 5.55 (s, 1H, CONH₂), 5.15 (s, 2H, ArCH₂O), 5.06 (s, 2H, ArCH₂O), 4.66-4.57 (m, 2H, NCH₂), 4.08-3.98 (m, 2H, OCH₂), 3.95-3.90 (m, 2H, ArCH₂), 3.79 (d, *J =* 13.8 Hz, 1H, ArCH₂), 3.44 (d, *J* = 12.1 Hz, 1H, ArCH₂), 3.21-3.15 (m, 1H, 1/2pyrrolidine-CH₂), 3.15-3.07 (m, 2H, pyrrolidine-CH), 3.05-2.95 (m, 1H, 1/2pyrrolidine-CH₂), 2.51-2.34 (m, 4H, pyrrolidine-CH₂), 2.25-2.16 (m, 2H, CH₂CH₂CH₂), 2.10 (s, 3H, ArCH₃), 1.95 (s, 3H, ArCH₃) 1.93-1.88 (m, 2H, pyrrolidine-CH₂), 1.81-1.71 (m, 4H, pyrrolidine-CH₂).

### Example 5

Synthesis of (4-((3'-(3-(4-(((carboxymethyl)amino)methyl)-1*H*-1,2,3-triazol-1-)propoxy)-2, 2'-dimethyl-[1,1'-biphenyl]-3-)methoxy)-5-chloro-2-((5-cyanopyridin-3-)methoxy)benzyl) glycine (II-5: R¹ and R² are 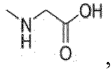 R³ and R⁴ is -CH₃, R⁵ is Cl, and R⁶ is 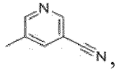 *n=3)*

Compound XVI-1 (0.20 g, 0.31 mmol) and glycine methyl ester hydrochloride (0.11 g, 0.95 mmol) were used as raw materials, the operation process was the same as that of compound II-1 to obtain 35 mg of yellow solid powder with a yield of 14.8 %. In methanol, saturation was conducted with hydrogen chloride in methanol solution to form a salt to obtain 39.56 mg of yellow solid powder with a yield of 98.9%.

¹H NMR (400 MHz, DMSO-*d*₆) δ 8.75 (d, *J=* 2.0 Hz, 1H, ArH), 8.72 (d, *J=* 2.3 Hz, 1H, ArH), 8.27 (t, *J* = 2.1 Hz, 1H, ArH), 8.16 (s, 1H, ArH), 7.51 (s, 1H, ArH), 7.49 (d, *J* = 7.6 Hz, 1H, ArH), 7.27 (t, *J* = 7.6 Hz, 1H, ArH), 7.21 (t, *J* = 8.0 Hz, 1H, ArH), 7.16 (s, 1H, ArH), 7.10 (d, *J* = 7.2 Hz, 1H, ArH), 6.94 (d, *J* = 7.6 Hz, 1H, ArH), 6.70 (d, *J* = 7.4 Hz, 1H, ArH), 5.31 (s, 4H, 2 ArCH₂O), 4.59 (t, *J* = 6.9 Hz, 2H, NCH₂), 4.12-4.08 (m, 2H, OCH₂), 4.02 (s, 2H, ArCH₂), 3.95 (s, 2H, ArCH₂), 3.22 (s, 2H, CH₂CO), 3.14 (s, 2H, CH₂CO), 2.34 (m, 2H, CH₂CH₂CH₂), 2.04 (s, 3H, ArCH₃), 1.82 (s, 3H, ArCH₃).

### Example 6

Synthesis of (4-((3'-(2-(4-(((1-carboxy-2-hydroxyethyl)amino)methyl)-1*H*-1,2,3-triazol-1-)ethyl oxy)-2,2'-dimethyl-[1,1'-biphenyl]-3-)methoxy)-5-chloro-2-((5-cyanopyridin-3-)methoxy)benzyl)serine (II-6: R¹ and R² are 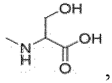 R³ and R⁴ are -CH3, R5 is Cl, R6 is 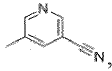 n=2)

### Synthesis of 1-bromo-3-(2-bromoethoxy)-2-methylbenzene (V-2)

Compound IV-1 (4.20 g, 18.3 mmol) and 1,2-dibromoethane (4.75 g, 54.9 mmol) were used as raw materials, the operation process was the same as that of compound V-1 to obtain 3.94 g of a light yellow solid with a yield of 73.7%.

MS (ESI): m/z [M+H]⁺. Calcd for C₉H₁₁OBr₂:291.9; found: 291.9.

¹H NMR (300 MHz, Chloroform-*d*) δ 7.24 (d, *J* = 8.1 Hz, 1H, ArH), 7.05 (t, *J* = 8.1 Hz, 1H, ArH), 6.80 (d, *J=* 8.2 Hz, 1H, ArH), 4.33 (t, J=6.1Hz, 2H, OCH₂), 3.71 (t, *J=* 6.0 Hz, 2H, CH₂Br), 2.39 (s, 3H, CH₃).

### 1-(2-azidoethoxy)-3-bromo-2-methylbenzene (VI-2)

Compound V-2 (5.00 g, 17.1 mmol) and TMSN₃ (2.69 g, 20.52 mmol) were used as raw materials, the operation process was the same as that of compound VI-1 to obtain 4.15 g of a colorless transparent liquid with a yield of 95.3%.

¹H NMR (300 MHz, Chloroform-*d*) δ 7.49 (d, *J*= 7.9Hz, 1H, ArH), 7.02 (t, *J* =7.5Hz, 1H, ArH), 6.79(d, *J* = 8.1 Hz, 1H, ArH), 4.25 (t, *J* = 5.8 Hz, 2H, OCH₂), 3.86 (t, *J* =5.9 Hz, 2H, CH₂N₃), 2.37 (s, 3H, CH₃).

### 1-(2-(3-bromo-2-methylphenoxy)ethyl)-4-(diethoxymethyl)-1H-1,2,3-triazole (VII-2)

Compound VI-2 (4.00 g, 15.6 mmol) and 3,3-diethoxy-1-propyne (2.00g, 15.6 mmol) were used as raw materials, the operation process was the same as that of compound VII-1 to obtain 4.32 g of a white solid. The solid was directly submitted to the next step without further processing.

### Synthesis of 1-(2-(3-bromo-2-methylphenoxy)ethyl)-1H-1,2,3-triazole-4-carbaldehyde (VIII-2)

Compound VII-2 (4.32 g, 11.2 mmol) was used as a raw material, the operation process was the same as that of compound VIII-1 to obtain 2.53 g of a white solid with a yield of 52.3% in the two steps. m.p at 102.0-104.0°C.

¹H NMR (300 MHz, Chloroform-*d*) δ 10.18 (s, 1H, CHO), 8.30 (s, 1H, ArH), 7.23 (d, *J=* 8.1 Hz, 1H, ArH), 7.02 (t, *J* = 8.2Hz, 1H, ArH), 6.73 (d, *J* = 8.1Hz, 1H, ArH), 4.91 (t, *J* =5. 1Hz, 2H, NCH₂), 4.42 (t, *J* = 5.1Hz, 2H, CH₂O), 2.27 (s, 3H, CH₃).

### 1-(2-(2-methyl-3-(4,4,5,5-tetramethyl-1,3,2-dioxaborolan-2-)phenoxy)ethyl)-1H-1,2,3-triazole-4-carbal dehyde (IX-2)

Compound VIII-2 (2.50 g, 8.06 mmol) was used as a raw material, the operation process was the same as that of compound IX-1 to obtain 2.45 g of white solid powder with a yield of 85.3 %. m.p at 106.5-109.0°C.

¹H NMR (300 MHz, Chloroform-*d*) δ 10.19 (s, 1H, CHO), 8.37 (s, 1H, ArH), 7.43 (dd, *J*= 7.5, 1.2 Hz, 1H, ArH), 7.17 (t, *J* = 7.8 Hz, 1H, ArH), 6.87 (dd, *J* = 8.2, 1.3 Hz, 1H, ArH), 4.91 (t, *J* = 5.4 Hz, 2H, NCH₂), 4.39 (t, *J*= 5.5 Hz, 2H, OCH₂), 2.40 (s, 3H, CH₃), 1.38 (s, 12H, (CH₃)₄).

### 1-(2-((3'-((2-chloro-4-formyl-5-hydroxyphenoxy)methyl)-2,2'-dimethyl-[1,1'-biphenyl]-3-)oxy)ethyl)-1 H-1,2,3-triazole-4-carbaldehyde (XV-2)

Compound XIV (1.00 g, 2.81 mmol) and compound IX-2 (1.21 g, 3.37 mmol) were used as raw materials, the operation process was the same as that of compound XV-1, and purification was conducted through column chromatography (petroleum ether:ethyl acetate = 2:1) to obtain 0.85 g of yellow solid powder with a yield of 59.7%, m.p. at 138.0-140.0°C .

¹H NMR (300 MHz, Chloroform-d) δ 11.48 (s, 1H, OH), 10.19 (s, 1H, CHO), 9.74 (s, 1H, CHO), 8.35 (s, 1H, ArH), 7.59 (s, 1H, ArH), 7.23 (t, *J* = 7.9 Hz, 1H, ArH), 7.14 (d, *J* = 7.6, Hz, 2H, ArH), 7.02 (t, *J* = 8.2 Hz, 1H, ArH), 6.67 (s, 1H, ArH), 6.54 (d, *J* = 8.1 Hz, 1H, ArH), 6.41 (d, *J* = 8.2 Hz, 1H, ArH), 5.24 (s, 2H, ArCH₂O), 4.95 (t, *J* = 4.9 Hz, 2H, NCH₂), 4.43-4.38 (t, *J* = 4.9 Hz, 2H, OCH₂), 2.09 (s, 3H, ArCH₃), 2.07 (s, 3H, ArCH₃).

### 5-((4-chloro-2-formyl-5-((3'-(2-(4-formyl-1H-1,2,3-triazol-1-)ethoxy)-2,2'-Dimethyl-[1,1'-biphenyl]-3-)met hoxy)phenoxy) methyl)nicotinonitrile (XVI-2)

Compound XV-2 (0.70 g, 1.38 mmol), 5-(chloromethyl) nicotinonitrile (0.31 g, 1.66 mmol), cesium carbonate (1.34 g, 4.14 mmol), potassium iodide (11 g, 0.07 mmol) and *N,N* - dimethylformamide (15 mL) were successively added into an eggplant-shaped flask, and the temperature was raised to 80°C for 6 hours of reaction. After the reaction of the raw materials was completed (monitored by TLC), heating was stopped, and then the solution was cooled to room temperature. The reaction solution was slowly poured into ice water, extraction was conducted with ethyl acetate (20 mL×3), the organic phases were combined, and washing was conducted with water (20 mL × 2) and saturated sodium chloride solution (20 mL × 2), respectively; then drying was conducted over anhydrous sodium sulfate, suction filtration was conducted, and the solvent was removed under reduced pressure to obtain a brownish black solid, which was purified by column chromatography (petroleum ether: ethyl acetate=15:1-8:1), 0.48 g of yellow solid powder was obtained with a yield of 55.8%. m.p. at 69.0-71.0°C.

¹H NMR (300 MHz, DMSO-*d*₆) δ 10.22 (s, 1H, CHO), 10.05 (s, 1H, CHO), 8.99 (s, 1H, ArH), 8.78 (d, *J* = 2.0 Hz, 1H, ArH), 8.76 (d, *J*= 2.3 Hz, 1H, ArH), 8.32 (t, *J*= 2.1 Hz, 1H, ArH), 7.75 (s, 1H, ArH), 7.54 (d, *J* = 7.0 Hz, 1H, ArH), 7.34-7.28 (m, 2H, ArH), 7.25 (t, *J* = 7.9 Hz, 1H, ArH), 7.09 (d, *J* = 6.2 Hz, 1H, ArH), 7.03 (d, *J* = 7.5 Hz, 1H, ArH), 6.74 (d, *J* = 7.3 Hz, 1H, ArH), 5.47 (s, 2H, ArCH₂O), 5.44 (s, 2H, ArCH₂O), 4.96 (t, *J* = 5.3 Hz, 2H, NCH₂), 4.57-4.51 (m, 2H, OCH₂), 2.03 (s, 3H, ArCH₃), 1.70 (s, 3H, ArCH₃).

### Synthesis of (4-((3'-(2-(4-(((1-carboxy-2-hydroxyethyl)amino)methyl)-1H-1,2,3-triazol-1-yl)ethoxy)-2,2'-dimethyl-[1,1'-biphenyl]-3-)methoxy)-5-chloro-2-((5-cyanopyridin-3-)methoxy)benzyl)serine (II-6)

Compound XVI-1 (0.30 g, 0.48 mmol) and serine methyl ester hydrochloride (0.27 mg, 1.93 mmol) were dissolved in dichloromethane (5 mL) and were successively added into a reaction flask, and stirred at a room temperature for 30 min, and NaBH(OAc)₃ (1.49 g, 7.72 mmol) was slowly added, and then reacted at a room temperature for 2 h. The reaction of raw materials was completed (monitored by TLC), and the reaction was stopped. The pH was adjusted to 7 with saturated sodium bicarbonate solution, extraction was conducted with dichloromethane (10 mL × 2), the organic phases were combined, washing was conducted with water (10 mL × 2) and saturated sodium chloride solution (10 mL × 2), respectively; and then drying was conducted over sodium sulfate, suction filtration was conducted, and the solvent was removed under reduced pressure to obtain a crude product, which was subjected to column chromatography (dichloromethane:methanol=100:1-40:1) to obtain 153 mg of a viscous colorless liquid. This was dissolved in methanol (1.0 mL) and tetrahydrofuran (1.0 mL) into an eggplant-shaped flask successively; then lithium hydroxide monohydrate (21 mg, 0.46 mmol) was added, and then the solution was allowed to react at a room temperature for 3 h. After the reaction of the raw materials was completed (monitored by TLC (dichloromethane:methyl alcohol=10:1)), the organic solvent was evaporated under reduced pressure, the pH of the residue was adjusted to 6-7 with 0.5 M NaOH, and a solid was precipitated, which was then filtered by suction, and dried to obtain 0.12 g of yellow solid powder with a yield of 31.1%.

¹H NMR (300 MHz, DMSO-*d*₆) δ 8.76 (d, *J*= 2.1 Hz, 1H, ArH), 8.53 (d, *J*= 2.2 Hz, 1H, ArH), 8.30 (s, 1H, ArH), 7.99 (d, *J* = 2.0 Hz, 1H, ArH), 7.69-7.46 (m, 3H, ArH), 7.33-7.15 (m, 3H, ArH), 7.07 (d, *J* = 7.6 Hz, 1H, ArH), 6.92 (d, *J* = 7.1 Hz, 1H, ArH), 5.31 (s, 2H, ArCH₂O), 5.18 (s, 2H ArCH₂O), 4.88 (s, 2H, OCH₂), 4.46 (t, *J* = 4.7 Hz, 2H, NCH₂), 4.24 (s, 2H, ArCH₂), 4.09 (dd, *J* = 13.7, 3.9 Hz, 2H, ArCH₂), 3.95-3.83 (m, 4H, CH₂OH), 3.46 (t, 1H, *J* = 3.7 Hz, CHCO₂H), 3.37 (t, 1H, *J* = 3.7 Hz, CHCO₂H), 2.44 (s, 3H, ArCH₃), 1.98 (s, 3H, ArCH₃).

### Example 7

Synthesis of (4-((3'-(3-(4-(((1-carboxy-2-hydroxyethyl)amino)methyl)-1*H*-1,2,3-triazol-1-)propoxy)-2,2'-dimethyl-[1,1'-b iphenyl]-3-yl)methoxy)-5-chloro-2-methoxybenzyl)serine (II-7 : R¹ and R² are 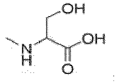 R³ and R⁴ are -CH₃, R⁵ is Cl, R⁶ is H, *n=3)*

### Synthesis of 1-(3-((3'-((2-chloro-4-formyl-5-methoxyphenoxy) methyl)-2,2'-dimethyl-[1,1'-biphenyl]-3-)oxy)propyl)-1H-1,2,3-triazole-4-carbaldehyde (XVI-3)

Compound XV-1 (0.72 g, 1.38 mmol), CH₃I (0.30 g, 2.07 mmol) and potassium carbonate (0.38 g, 2.76 mmol) were dissolved in *N*,*N*-dimethyl Formamide (10 mL) and then reacted at room temperature for 6 h. After the reaction of the raw materials was completed (monitored by TLC), heating was stopped, and then cooled to a room temperature. The reaction solution was slowly poured into ice water (30 mL), extraction was conducted with ethyl acetate (10 mL×3), the organic phases were combined, and washing was conducted with water (10 mL × 2) and saturated sodium chloride solution (10 mL × 2), respectively; and then drying was conducted over anhydrous sodium sulfate, suction filtration was conducted, and the solvent was removed under reduced pressure to obtain a crude product, which was purified by column chromatography (petroleum ether:ethyl acetate=8:1-4:1) to obtain 0.53 g of white solid powder with a yield of 71.7 %.

¹H NMR (300 MHz, DMSO-*d*₆) δ 10.19 (s, 1H, CHO), 10.04 (s, 1H, CHO), 9.00 (s, 1H, ArH), 7.71 (s, 1H, ArH), 7.57 (d, *J* = 6.2 Hz, 1H, ArH), 7.34 (t, *J* = 7.8 Hz, 1H, ArH), 7.23 (t, *J* = 7.9 Hz, 1H, ArH), 7.15 (s, 1H, ArH), 7.12 (d, *J* = 6.0 Hz, 1H, ArH), 6.96 (d, *J* = 8.4 Hz, 1H, ArH), 6.72 (d, *J* = 7.6 Hz, 1H, ArH), 5.47 (s, 2H, CH₂O), 4.71 (t, *J*= 6.8 Hz, 2H, NCH₂CH₂), 4.14-4.05 (m, 2H, OCH₂), 4.03 (s, 3H, OCH₃), 2.43 (t, *J* = 6.6 Hz, 2H, CH₂CH₂CH₂), 2.06 (s, 3H, ArCH₃), 1.81 (s, 3H, ArCH₃).

### Synthesis of (4-((3'-(3-(4-(((1-carboxy-2-hydroxyethyl)amino)methyl)-1H-1,2,3-triazol-1-yl)propane Oxy)-2,2'-dimethyl-[1,1'-biphenyl]-3-yl)methoxy)-5-chloro-2-methoxybenzyl)serine (II-7)

Compound XVI-3 (250 g, 0.47 mmol) and serine methyl ester hydrochloride (182 mg, 1.17 mmol) were used as raw materials, the operation process was the same as that of compound II-1 to obtain 42 mg of brown solid powder with a yield of 12.6 %.

¹H NMR (300 MHz, DMSO-*d*₆) δ 8.19 (d, *J* = 2.8 Hz, 1H, ArH), 7.64 (d, *J*= 5.4 Hz, 1H, ArH), 7.50 (dd, *J* = 11.0, 6.7 Hz, 1H, ArH), 7.33-7.13 (m, 2H, ArH), 7.12-6.99 (m, 2H, ArH), 6.93 (d, *J* = 8.1 Hz, 1H, ArH), 6.66 (t, *J* = 7.5 Hz, 1H, ArH), 5.34 (s, 2H, ArCH₂O), 4.61 (t, *J* = 7.0 Hz, 2H, NCH₂), 4.13-3.93 (m, 6H, CH₂OH/ArCH₂ /OCH₂), 3.89 (s, 3H, OCH₃), 3.81-3.69 (m, 2H, CH₂OH), 3.66 (s, 2H, ArCH₂), 3.25-3.12 (m, 2H, CHCO₂H), 2.40-2.21 (m, 2H, CH₂CH₂CH₂), 2.03 (s, 3H, ArCH₃), 1.83 (s, 3H, ArCH₃).

### Example 8

Synthesis of 1-(4-((3'-(3-(4-((2-carbamoylpyrrolidin-1-)methyl)-1H-1,2,3-triazol-1-) Propoxy)-2,2'-dimethyl-[1,1'-biphenyl]-3-)methoxy)-5-chloro-2-methoxybenzyl)pyrrolidine-2-formamide (II-8: R¹ and R² are 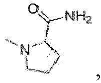 R³ and R⁴ are -CH₃, R⁵ is Cl, R⁶ is H, *n=3)*

Compound XVI-3 (250 g, 0.47 mmol) and L-prolineamide (134 mg, 1.17 mmol) were used as raw materials, the operation process was the same as that of compound II-4 to obtain 49 mg of brown solid powder with a yield of 14.3 %.

¹H NMR (400 MHz, Chloroform-*d*) δ 7.64 (br, 1H, 1/2CONH₂), 7.51 (d, *J* = 7.4 Hz, 1H, ArH), 7.47 (s, 1H, ArH), 7.38-7.26 (m, 1H, 1/2CONH₂), 7.25 (s, 1H, ArH), 7.24-7.17 (m, 2H, ArH), 7.14 (d, *J* = 6.8 Hz, 1H, ArH), 6.82 (d, *J=* 8.1 Hz, 1H, ArH), 6.79 (d, *J=* 7.5 Hz, 1H, ArH), 6.59 (s, 1H, ArH), 5.95-5.76 (br, 1H, 1/2CONH₂), 5.66 (br, 1H, 1/2CONH₂), 5.20 (s, 2H, ArCH₂O), 4.63 (t, *J*= 6.4 Hz, 2H, NCH₂), 4.09-4.00 (m, 2H, OCH₂), 3.99-3.93 (m, 2H, ArCH₂N), 3.82 (s, 3H, OCH₃), 3.35-3.25 (m, 1H, CHCO), 3.22-3.09 (m, 2H, ArCH₂N), 3.00-2.91 (m, 1H, CHCO), 2.52-2.43 (m, 4H, 2CH₂-prolinamide), 2.35-2.18 (m, 4H, 2CH₂-prolinamide&CH₂CH₂CH₂), 2.16 (s, 3H, ArCH₃), 2.02-1.91 (m, 5H, ArCH₃, CH₂-prolinamide), 1.82-1.70 (m, 4H, CH₂-prolinamide).

HRMS (ESI): *m*/*z* [M+Na]⁺calculated for C₃₉H₄₉ClN₇O₅, 752.3303; found:752.3301.

### Example 9

Synthesis of 2-(((1-(3-((3'-((2-chloro-4-(((2-hydroxyethyl)amino) methyl)-5-methoxyphenoxy)methyl)-2,2'-dimethyl-[1,1'-biphenyl]-3-)oxy)propyl)-1*H*-1,2,3-triazol-4-)meth yl)ethyl-1-ol (II-9: R¹ and R² are 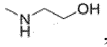 , R³ and R⁴ are -CH₃, R⁵ is Cl, R⁶ is H, *n*=3)

Compound XVI-3 (200 g, 0.38 mmol) and ethanolamine (69.4 mg, 1.12 mmol) were used as raw materials, the operation process was the same as that of compound II-2 to obtain 35 mg of yellow solid powder with a yield of 14.8 %.

¹H NMR (300 MHz, Chloroform-*d*) δ 7.61 (d, *J* = 7.7 Hz, 1H, ArH), 7.57 (s, 1H, ArH), 7.54 (d, *J*= 7.7 Hz, 1H, ArH), 7.47 (d, *J* = 7.7 Hz, 1H, ArH), 7.39 (s, 1H, ArH), 7.27-7.10 (m, 2H, ArH), 6.84 (t, *J* = 7.8 Hz, 2H, ArH), 6.62 (s, 1H, NH), 6.57 (s, 1H, NH), 5.24 (s, 2H, ArCH₂O), 5.18 (s, 2H, OH), 4.67 (t, *J* = 6.8 Hz, 2H, NCH₂), 4.12-4.04 (m, 2H, OCH₂), 3.98 (s, 2H, ArCH₂N), 3.88 (s, 3H, OCH₃), 3.86 (s, 2H, ArCH₂N), 3.72-3.69 (m, 4H, NHCH₂CH₂), 3.54 (s, 2H, NH), 2.87 (t, *J* = 5.3 Hz, 4H, NHCH₂CH₂), 2.51-2.46 (m, 2H, CH₂CH₂CH₂) 2.14 (s, 3H, ArCH₃), 1.96 (s, 3H, ArCH₃).

HRMS (ESI): *m*/*z* [M+Na]⁺calculated for C₃₃H₄₃ClN₅O₅, 646.2772; found: 646.2778.

### Example 10

Synthesis of *N-*(2-((4-((3'-(3-(4-(((2-acetylaminoethyl)amino)methyl)-1*H*-1,2,3-triazole-1-)propoxy)-2,2'-dimethyl-[1,1'-biphenyl]-3-)methoxy)-5-chloro-2-methoxybenzyl)amino)ethyl)acetamide (II-10: R¹ and R² are 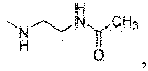 R³ and R⁴ are -CH₃, R⁵ is Cl, R⁶ is H, *n*=3)

Compound XVI-3 (200 g, 0.37 mmol) and *N*-acetylethylenediamine(151 mg, 1.48 mmol) were used as raw materials, the operation process was the same as that of compound II-3 to obtain 72 mg of yellow solid powder with a yield of 27.2 %, m.p. at 48.0-50.0 °C.

¹H NMR (300 MHz, DMSO-*d*₆) δ 8.11 (s, 1H, CONH), 8.02 (s, 1H, CONH), 7.54 (d, *J* = 8.2 Hz, 1H, ArH), 7.45 (s, 1H, ArH), 7.35 (s, 1H, ArH), 7.28 (d, *J* = 7.6 Hz, 1H, ArH), 7.25-7.20 (m, 1H, ArH), 7.08-7.01 (m, 1H, ArH), 7.00-6.95 (m, 2H, ArH), 6.71 (d, *J* = 8.0 Hz, 1H, ArH), 5.30 (s, 2H, ArCH₂O), 4.59-4.55 (m, 2H, NCH₂), 4.10-4.00 (m, 2H, OCH₂), 3.85 (s, 3H, OCH₃), 3.83 (s, 2H, ArCH₂N), 3.74 (s, 2H, ArCH₂N), 3.60 (s, 1H, NH), 3.51 (s, 1H, NH), 3.20-3.12 (m, 4H, CONHCH₂), 2.60-2.48 (m, 2H, CH₂CH₂CH₂), 2.40-2.25 (m, 4H, NHCH₂), 2.06 (s, 3H, ArCH₃), 1.87 (s, 3H, ArCH₃), 1.81 (s, 6H, COCH₃).

HRMS (ESI): *m*/*z* [M+H]⁺ calculated for C₃₇H₄₉ClN₇O₅, 706.3484; found: 706.3477.

### Example 11

Synthesis of (R)-1-((1-(3-((3'-((2-chloro-4-(((R)-3-hydroxypyrrolidin-1-yl)methyl)-5-methoxy phenoxy)methyl)-2,2'-dimethyl-[1,1'-biphenyl]-3-)oxy)propyl)-1*H*-1,2,3-triazol-4-)methyl)pyrrolidin-3-ol (II-11: R¹ and R² are 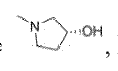 , R³ and R⁴ are -CH₃, R⁵ is Cl, R⁶ is H, *n*=3)

Compound XVI-3 (300 g, 0.56 mmol) and R-3-pyrrolidinol hydrochloride (0.35 mg, 2.81 mmol) were used as raw materials, the operation process was the same as that of compound II-3 to obtain 120 mg of white solid powder with a yield of 32.9 %.

¹H NMR (300 MHz, Chloroform-*d*) δ 7.69 (s, 1H, ArH), 7.53 (d, *J*= 7.6 Hz, 1H, ArH), 7.42 (s, 1H, ArH), 7.33 (s, 1H, ArH), 7.22 (t, *J*= 7.8 Hz, 1H, ArH), 7.17 (d, *J* = 7.3 Hz, 1H, ArH), 6.83 (t, *J* = 7.8 Hz, 2H, ArH), 6.63 (s, 1H, ArH), 5.25 (s, 2H, ArCH₂O), 4.67 (t, *J* = 6.8 Hz, 2H, NCH₂), 4.55-4.32 (m, 2H, OCH₂), 4.13-3.99 (m, 2H, CHOH), 3.94 (s, 2H, ArCH₂N), 3.87 (s, 3H, OCH₃), 3.86 (s, 2H, ArCH₂N), 3.21 (q, *J* = 9.2 Hz, 1H, pyrrolidine-CH₂), 3.13-2.98 (m, 2H, pyrrolidine-CH₂), 2.94-2.80 (m, 2H, pyrrolidine-CH₂), 2.79-2.70 (m, 1H, pyrrolidine-CH₂), 2.70-2.55 (m, 2H, pyrrolidine-CH₂), 2.54-2.44 (m, 2H, CH₂CH₂CH₂), 2.36-2.17 (m, 2H, pyrrolidine-CH₂), 2.14 (s, 3H, ArCH₃), 1.96 (s, 3H, ArCH₃), 1.93-1.79 (m, 2H, pyrrolidine-CH₂).

HRMS (ESI): *m*/*z* [M+H]⁺calculated for C₃₇H₄₇ClN₅O₅, 676.3266; found: 676.3256.

### Example 12

Synthesis of 3-((4-((3'-(3-(4-(((2-carboxyethyl)amino)methyl)-1*H*-1,2,3-triazol-1-)propoxyl)-2,2'-dimethyl-[1,1'-biphenyl] -3-)methoxy)-5-chloro-2-methoxybenzyl)amino)propionic acid (II-12: R¹ and R² are 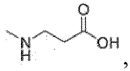 R³ and R⁴ are -CH₃, R⁵ is Cl, R⁶ is H, *n=3)*

Compound XVI-3 (210 g, 0.39 mmol) and β-alanine ethyl ester hydrochloride (0.52 mg, 1.57 mmol) were used as raw materials, the operation process was the same as that of compound II-7 to obtain 157 mg of yellow solid powder with a yield of 57.6 %.

¹H NMR (300 MHz, DMSO-*d*₆+D₂O) δ 8.20 (s, 1H, ArH), 7.54 (d, *J=* 7.5 Hz, 1H, ArH), 7.51 (s, 1H, ArH), 7.31 (t, *J*= 7.5 Hz, 1H, ArH), 7.23 (t, *J*= 8.0 Hz, 1H, ArH), 7.10 (d, *J* = 7.7 Hz, 1H, ArH), 7.04 (s, 1H, ArH), 6.96 (d, *J=* 8.1 Hz, 1H, ArH), 6.72 (d, *J=* 7.5 Hz, 1H, ArH), 5.36 (s, 2H, ArCH₂O), 4.62 (t, *J* = 7.0 Hz, 2H, NCH₂), 4.11-4.03 (m, 2H, OCH₂), 4.02 (s, 2H, ArCH₂N), 3.93 (s, 2H, ArCH₂N), 3.91 (s, 3H, OCH₃), 3.00-2.88 (m, 4H, NHCH₂), 2.50-2.42 (m, 4H, CH₂CO₂H& CH₂CH₂CH₂), 2.38-2.35 (m, 2H, CH₂CO₂H), 2.06 (s, 3H, ArCH₃), 1.84 (s, 3H, ArCH₃).

HRMS (ESI): *m*/*z* [M+H]⁺, calculated for C₃₅H₄₃ClN₅O₇ 680.2851; Found: 680.2834.

### Example 13

Synthesis of 1-(4-((3'-(3-(4-((2-carboxypiperidin-1-)methyl)-1*H*-1,2,3-triazol-1-)propoxyl)-2,2'-dimethyl-[1,1'-biphenyl]-3-)methoxy)-5-chloro-2-methoxybenzyl)piperidine-2-carboxy acid (II-13: R¹ and R² are 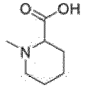 R³ and R⁴ are -CH₃, R⁵ is Cl, R⁶ is H, *n=3)*

Compound XVI-3 (300 g, 0.56 mmol) and methyl piperidine-2-carboxylate, hydrochloride (0.43 mg, 2.81 mmol) were used as raw materials, the operation process was the same as that of compound II-7 to obtain 106 mg of yellow solid powder with a yield of 24.8 %.

¹H NMR (400 MHz, Chloroform-*d*) δ 8.10 (s, 1H, ArH), 7.68 (s, 1H, ArH), 7.55 (dd, *J* = 7.7, 1.4 Hz, 1H, ArH), 7.31 (d, *J* = 15.1 Hz, 1H, ArH), 7.20 (t, *J*= 7.9 Hz, 1H, ArH), 7.13 (s, 1H, ArH), 7.10 (dd, *J* = 7.6, 1.4 Hz, 1H, ArH), 6.93 (d, *J* = 7.8 Hz, 1H, ArH), 6.70 (d, *J* = 7.3 Hz, 1H, ArH), 5.46 (s, 2H, ArCH₂O), 4.59 (t, *J*= 6.9 Hz, 2H, NCH₂), 4.01 (s, 3H, OCH₃), 3.79-3.56 (m, 4H, ArCH₂/ OCH₂), 3.53 (s, 2H, ArCH₂), 3.08 -2.97 (m, 2H, CH), 2.47-2.39 (m, 2H, CH₂CH₂CH₂), 2.36-2.29 (m, 2H, piperidine -CH₂), 2.04 (s, 3H, ArCH₃), 1.84 (s, 3H, ArCH₃), 1.72-1.61 (m, 4H, piperidine-CH₂), 1.57-1.57 (m, 4H, piperidine-CH₂), 1.38 -1.28 (m, 4H, piperidine-CH₂).

HRMS (ESI): *m*/*z* [M+H]⁺, calculated for C₄₁H₅₁ClN₅O₇, 760.3477; found: 760.3482.

### Example 14

Synthesis of (R)-1-((1-(3-((3'-((2-chloro-4-(((R)-3-hydroxypiperidin-1-)methyl)-5-methoxyl phenoxy)methyl)-2,2'-dimethyl-[1,1'-biphenyl]-3-)oxy)propyl)-1*H*-1,2,3-triazol-4-)methyl)piperidin-3-ol (II-14: R¹ and R² are 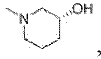 R³ and R⁴ are -CH₃, R⁵ is Cl, R⁶ is H, *n*=3)

Compound XVI-3 (200 g, 0.37 mmol) and R-piperidine-2-carboxylic acid methyl ester hydrochloride (0.23 mg, 1.50 mmol) were used as raw materials, the operation process was the same as that of compound II-3 to obtain 179 mg of yellow solid powder with a yield of 67.86 %.

¹H NMR (400 MHz, Chloroform-*d*) δ 7.52 (s, 1H, ArH), 7.34 (d, *J* = 7.6 Hz, 1H, ArH), 7.42 (s, 1H, ArH), 7.20 (s, 1H, ArH), 7.22 (t, *J* = 8.0 Hz, 1H, ArH), 7.15 (d, *J* = 7.4 Hz, 1H, ArH), 6.81 (t, *J* = 7.7 Hz, 2H, ArH), 6.59 (s, 1H, ArH), 5.20 (s, 2H, ArCH₂O), 4.65 (t, *J* = 7.1 Hz, 2H, NCH₂), 4.06-4.01 (m, 2H, OCH₂), 3.91-3.86 (m, 2H, CHOH), 3.85 (s, 2H, ArCH₂N), 3.82 (s, 3H, OCH₃), 3.73 (s, 2H, ArCH₂N), 3.55 (q, *J =* 9.2 Hz, 1H, pyrrolidine-CH₂), 2.63-2.53 (m, 2H, pyrrolidine-CH₂), 2.94-2.49 (m, 2H, pyrrolidine-CH₂), 6.9-2.44 (m, 1H, pyrrolidine-CH₂), 2.70-2.28 (m, 2H, pyrrolidine-CH₂), 2.13-2.44 (m, 2H, CH₂CH₂CH₂), 1.96-2.17 (m, 2H, pyrrolidine-CH₂), 2.14 (s, 3H, ArCH₃), 1.89 (s, 3H, ArCH₃), 1.69-1.49 (m, 2H, pyrrolidine-CH₂).

### Example 15

(4-((3'-(3-(4-(((1-carboxyethyl)amino)methyl)-1*H*-1,2,3-triazol-1-yl)propoxy)-2,2'-dimethyl-[1,1'-biph enyl]-3-)methoxy)-5-chloro-2-methoxybenzyl)alanine (II-15: R¹ and R² are 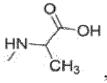 , R³ and R⁴ are -CH₃, R⁵ is Cl, R⁶ is H, *n*=3)

Compound XVI-3 (210 g, 0.39 mmol) and β-alanine ethyl ester hydrochloride (241 mg, 1.57 mmol) were used as raw materials, the operation process was the same as that of compound 11-3, and purified by column chromatography (petroleum ether: Methanol=80:1-30:1) to obtain 157 mg of brown yellow solid powder with a yield of 57.6%.

¹H NMR (300 MHz, DMSO-*d*₆) *δ* 8.27 (s, 1H, ArH), 7.56 (s, 1H, ArH), 7.52 (d, *J*= 7.2 Hz, 1H, ArH), 7.28 (t, *J* = 7.7 Hz, 1H, ArH), 7.21 (t, J= 7.8 Hz, 1H, ArH), 7.07 (d, *J* = 6.5 Hz, 1H, ArH), 7.01 (s, 1H, ArH), 6.94 (d, *J* = 8.3 Hz, 1H, ArH), 6.69 (d, *J*= 7.5 Hz, 1H, ArH), 5.35 (s, 2H, ArOCH₂), 4.61 (t, *J=* 7.0 Hz, 2H, NCH₂), 4.22-4.07 (m, 2H, OCH₂), 3.87 (s, 3H, OCH₃), 4.08-4.00 (m, 6H, ArCH₂&CHCH₃), 2.41-2.25 (m, 2H, CH₂CH₂CH₂), 2.03 (s, 3H, ArCH₃), 1.80 (s, 3H, ArCH₃), 1.38 (t, *J*= 6.8 Hz, 6H, CH₃).

HRMS (ESI): *m*/*z* [M+H]⁺, calculated for C₃₅H₄₃ClN₅O₇, 680.2851; found: 680.28394

### Example 16

Synthesis of (4-((3'-(3-(4-(((1-carboxy-2-hydroxyethyl)amino)methyl)-1*H*-1,2,3-triazol-1-)propoxyl)-2,2'-dichloro-[1,1'-b iphenyl]-3-)methoxy)-5-chloro-2-((5-cyanopyridin-3-)methoxy)benzyl)serine (II-16: R¹ and R² are 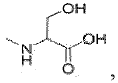 R³ and R⁴ are -CH₃, R⁵ is Cl, R⁶ is 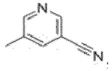 *n=3)*

### Synthesis of 1-bromo-3-(3-bromopropoxy)-2-chlorobenzene (V-3)

3-Bromo-2-chlorophenol (10.00 g, 48.2 mmol) and 1,3-dibromopropane (29.40 mg, 144.6 mmol) were used as raw materials, the operation process was the same as that of compound V-1, and purification was conducted through column chromatography to obtain 14.57 of colorless and a transparent oily liquid with a yield of 92.0%.

¹H NMR (300 MHz, Chloroform-*d*) *δ* 7.26 (dd, *J=* 8.1, 1.4 Hz, 1H, ArH), 7.10 (t, *J* = 8.2 Hz, 1H, ArH), 6.91 (dd, *J* = 8.3, 1.4 Hz, 1H, ArH), 4.19 (t, *J* = 5.7 Hz, 2H, OCH₂), 3.69 (t, *J* = 6.3 Hz, 2H, CH₂Br), 2.47 - 2.31 (m, 2H, CH₂CH₂CH₂).

### Synthesis of 1-(3-azidopropoxy)-3-bromo-2-chlorobenzene (VI-3)

Compound V-3 (10.00 g, 30.7 mmol) and TMSN₃ (4.84 mL, 36.8 mmol) were used as raw materials, the operation process was the same as that of compound V-1, and purification was conducted through column chromatography to obtain 8.62 of colorless and transparent oily liquid with a yield of 98.3%.

¹H NMR (300 MHz, DMSO-*d*₆) *δ* 7.37 (dd, *J* = 7.8, 1.7 Hz, 1H, ArH), 7.28 (t, *J* = 8.0 Hz, 1H, ArH), 7.21 (dd, *J* = 8.2, 1.7 Hz, 1H, ArH), 4.18 (t, *J* = 6.1 Hz, 2H, OCH₂), 3.57 (t, *J* = 6.7 Hz, 2H, NCH₂), 2.04 (p, *J* = 6.4 Hz, 2H, CH₂CH₂CH₂).

### Synthesis of 1-(3-(3-bromo-2-chlorophenoxy)propyl)-4-(diethoxymethyl)-1H-1,2,3-triazole (VII-3)

Compound VI-3 (4.82 g, 16.6 mmol) and propionaldehyde diethyl acetal (2.34 mL, 18.26 mmol) were used as raw materials, the operation process was the same as that of compound VII-1, and purification was conducted through column chromatography to obtain 6.68 g of colorless and transparent oily liquid, which was directly used for the next reaction.

### Synthesis of 1-(3-(3-bromo-2-chlorophenoxy)propyl)-1H-1,2,3-triazole-4-carbaldehyde (VIII-3)

Compound VII-3 (6.68 g, 16.0 mmol) and trifluoroacetic acid (7 mL) were used as raw materials, the operation process was the same as that of compound VIII-1 to obtain 4.17 g of white solid powder with a yield of 75.9%. m.p at 112-114 °C.

¹H NMR (300 MHz, DMSO-*d*₆) *δ* 10.05 (s, 1H, CHO), 8.98 (s, 1H, ArH), 7.37 (dd, *J=* 8.0, 1.5 Hz, 1H, ArH), 7.27 (t, *J* = 8.1 Hz, 1H, ArH), 7.18 (dd, *J* = 8.3, 1.6 Hz, 1H, ArH), 4.69 (t, *J* = 6.9 Hz, 2H, NCH₂), 4.14 (t, *J* = 5.9 Hz, 2H, OCH₂), 2.42 (p, *J* = 6.8 Hz, 2H, CH₂CH₂CH₂).

Synthesis of 1-(3-(2-chloro-3-(4,4,5,5-tetramethyl-1,3,2-dioxolan-2-yl)phenoxy)propyl)-1H-1,2,3-triazole-4-carbaldehyde (IX-3)

Compound VIII-3 (6.42 g, 18.6 mmol) and B₂Pin₂ (5.68 g, 22.36 mmol) were used as raw materials, the operation process was the same as that of compound IX-1 to obtain 5.07 g of light yellow solid powder with a yield of 75.6%, m.p. at 53.0-54.0 °C.

¹H NMR (300 MHz, Chloroform-*d*) *δ* 10.17 (s, 1H, CHO), 8.20 (s, 1H, ArH), 7.42 (dd, *J* = 7.9, 1.7 Hz, 1H, ArH), 7.25 (td, *J* = 7.8, 1.7 Hz, 1H, ArH), 6.91 (dd, *J* = 8.2, 1.5 Hz, 1H, ArH), 4.79 (t, *J* = 6.7 Hz, 2H, NCH₂), 4.06 (t, *J*= 5.6 Hz, 2H, OCH₂), 2.55 (p, *J*= 6.6 Hz, 2H, CH₂CH₂CH₂), 1.96 (s, 12H, CH₃).

### Synthesis of methyl 3-bromo-2-chlorobenzoate (XI-2)

3-Bromo-2-chlorobenzoic acid (5.22 g, 22.17 mmol) and SOCl₂ (1.93 mL, 26.6 mmol) were used as raw materials, the operation process was the same as that of compound XI-1 to obtain 5.49 of colorless and a transparent viscous liquid with a yield of 99.3%.

¹H NMR (300 MHz, DMSO-*d*₆) *δ* 8.01 (dd, *J* = 8.1, 1.5 Hz, 1H, ArH), 7.79 (dd, *J* = 7.7, 1.6 Hz, 1H, ArH), 7.43 (t, *J=* 7.9 Hz, 1H, ArH), 3.91 (s, 3H, OCH₃).

### Synthesis of (3-bromo-2-chlorophenyl)methanol (XII-2)

Compound XI-2 (12.84 g, 51.5 mmol) and LiAlH₄ (2.35 g, 61.8 mmol) were used as raw materials, the operation process was the same as that of compound XII-1 to obtain 7.80 g of a colorless transparent viscous liquid with a yield of 68.4%.

¹H NMR (300 MHz, DMSO-*d*₆) *δ* 7.70 (dd, *J* = 7.9, 1.7 Hz, 1H, ArH), 7.64 - 7.55 (m, 1H, ArH), 7.34 (t, *J* = 7.8 Hz, 1H, ArH), 5.60 (t, *J* = 5.6 Hz, 1H, CH₂OH), 4.61 (d, *J* = 5.0 Hz, 2H, CH₂OH).

### Synthesis of 1-bromo-3-(bromomethyl)-2-chlorobenzene (XIII-2)

Compound XII-2 (6.40 g, 28.9 mmol) and PBr₃ (14.37 g, 43.34 mmol) were used as raw materials, the operation process was the same as that of compound XIII-1 to obtain 8.15 g of a colorless transparent liquid with a yield of 93.6%.

¹H NMR (300 MHz, Chloroform-*d*) *δ* 7.59 (dd, *J* = 8.0, 1.5 Hz, ArH), 7.39 (dd, *J* = 7.7, 1.6 Hz, ArH), 7.12 (t, *J* = 7.8 Hz, ArH), 4.61 (s, 2H, CH₂).

### Synthesis of 4-((3-bromo-2-chlorobenzyl)-oxyl)-5-chloro-2-hydroxybenzaldehyde (XIV-2)

Compound XIII-2 (5.00 g, 17.6 mmol) and 5-chloro-2,4-dihydroxybenzaldehyde (3.03 g, 17.6 mmol) were used as raw materials, the operation process was the same as that of compound XIV-1 to obtain 2.35 g of white powder with a yield of 35.5%. m.p. at 158.0-160.0°C.

¹H NMR (300 MHz, Chloroform-*d*) *δ* 11.41 (s, 1H, OH), 9. 71 (s, 1H, CHO), 7.62 (d, *J* = 6.5 Hz, 1H, ArH), 7.56 (s, 1H, ArH), 7.42 (dd, *J* = 7.1, 2.2 Hz, 1H, ArH), 7.34-7.26 (m, 1H, ArH), 6.58 (s, 1H, ArH), 5.27 (s, 2H, CH₂).

### Synthesis of 5-((4-chloro-5-((2,2'-dichloro-3'-(3-(4-formyl-1H-1,2,3-triazol-1-)propoxy-[1,1'-biphenyl]-3-)methoxy)-2-fo rmylphenoxy)methyl)nicotinonitrile (XVI-4)

### Synthesis of 5-((5-((3-bromo-2-chlorobenzyl)oxy)-4-chloro-2-formylphenoxy)methyl)nicotinonitrile (1)

Compound XIV-2 (1.00 g, 2.66 mmol), 5-(Chloromethyl)nicotinonitrile (0.60 g, 3.19 mmol), K₂CO₃ (1.10 g, 7.98 mmol), KI (0.02 g, 0.13 mmol) and DMF (10 mL) were successively added into an eggplant-shaped flask, and the temperature was raised to 80°C for 6 hours of reaction. After the reaction of raw materials was completed (monitored by TLC (petroleum ether:ethyl acetate=1:1)), heating was stopped, and then the solution was cooled to a room temperature. This was diluted with ice-cold water (20 mL), the mixture was extracted with ethyl acetate (20 mL×3), washed with saturated sodium chloride solution (20 mL × 3), and then dried over anhydrous magnesium sulphate. The solid is removed by suction filtration, and the solvent was evaporated under reduced pressure to obtain a crude product, which was purified by column chromatography to obtain 0.60 g of yellow solid powder with a yield of 46.1%. m.p. at 140.0-142.0°C.

¹H NMR (400 MHz, DMSO-*d*₆) *δ* 10.20 (s, 1H, CHO), 8.75 (d, *J* = 2.0 Hz, 1H, ArH), 8.73 (d, *J* = 2.3 Hz, 1H, ArH), 8.28 (t, *J* = 2.1 Hz, 1H, ArH), 7.73 (s, 1H, ArH), 7.71-7.65 (m, 1H, ArH), 7.59-7.54 (m, 1H, ArH), 7.46-7.43 (m, 1H, ArH), 7.25 (s, 1H, ArH), 5.45 (s, 2H, OCH₂), 5.42 (s, 2H, OCH₂).

### Synthesis of 5-((4-chloro-5-((2,2'-dichloro-3'-(3-(4-fonnyl-1H-1,2,3-triazol-1-)propoxy-[1,1'-biphenyl]-3-)methoxy)-2-fo rmylphenoxy)methyl)nicotinonitrile (XVI-4)

Compound IX-2 (1.29 g, 2.62 mmol) and compound 1 (1.23 g, 3.14 mmol) were used as raw materials, the operation process was the same as that of compound XVI-1 to obtain 0.87 g of yellowish white solid powder with a yield of 49.0%. m.p. at 58.0-60.0°C.

¹H NMR (300 MHz, DMSO-*d*₆) *δ* 10.24 (s, 1H, CHO), 10.05 (s, 1H, CHO), 8.99 (s, 1H, ArH), 8.82 (s, 1H, ArH), 8.64 (s, 1H, ArH), 8.09 (s, 1H, ArH), 7.76-7.70 (m, 2H, ArH), 7.60-7.54 (m, 1H, ArH), 7.49-7.42 (m, 2H, ArH), 7.34 (s, 1H, ArH), 7.25 (d, *J* = 8.8 Hz, 1H, ArH), 7.09 (d, *J* = 7.8 Hz, 1H, ArH), 5.55 (s, 2H, CH₂O), 5.44 (s, 2H, CH₂O), 4.71 (t, *J* = 6.7 Hz, 2H, NCH₂CH₂), 4.18 (t, *J* = 6.0 Hz, 2H, OCH₂), 2.49-2.29 (m, 2H, CH₂CH₂CH₂).

### Synthesis of (4-((3'-(3-(4-(((1-carboxy-2-hydroxyethyl)amino) methyl)-1H-1,2,3-triazol-1-)propoxyl)-2,2'-dichloro-[1,1'-biphenyl]-3-)methoxy)-5-chloro-2-((5-cyanopyridi n-3-) methoxy)benzyl)serine (II-15)

Compound XVI-4 (200 g, 0.30 mmol) and serine methyl ester hydrochloride (184 mg, 1.18 mmol) were used as raw materials, the operation process was the same as that of compound II-1 to obtain 83 mg of brownish solid powder with a yield of 32.9%.

¹H NMR (300 MHz, DMSO-*d*₆) *δ* 8.81 (s, 1H, ArH), 8.63 (s, 1H, ArH), 8.17 (s, 1H, ArH), 8.11 (t, *J*= 2.1 Hz, 1H, ArH), 7.72-7.66 (m, 1H, ArH), 7.58-7.52 (m, 1H, ArH), 7.48-7.38 (m, 3H, ArH), 7.25-7.18 (m, 2H, ArH), 7.11 (d, *J* = 7.5 Hz, 1H, ArH), 5.40 (d, *J*= 5.5 Hz, 2H, ArCH₂O), 5.29 (s, 2H, ArCH₂O), 4.61 (t, *J* = 6.8 Hz, 2H, NCH₂), 4.13-4.11 (m, 2H, ArCH₂N), 4.10-4.05 (m, 2H, OCH₂), 4.02 (s, 2H, ArCH₂N), 3.77-3.61 (m, 4H, 2CH₂OH), 3.27 (t, *J* = 5.1 Hz, 1H, CHCO₂H), 3.21 (t, *J* = 5.2 Hz, 1H, CHCO₂H), 2.39-2.35 (m, 2H, CH₂CH₂CH₂).

### Example 17

Synthesis of (4-((3'-(3-(4-(((carboxymethyl)amino)methyl)-1*H*-1,2,3-triazol-1-)propoxy)-2, 2'-dichloro-[1,1'-biphenyl]-3-)methoxy)-5-chloro-2-((5-cyanopyridin-3-)methoxy)benzyl) glycine (II-17: R¹ and R² are 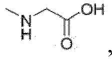 R³ and R⁴ are -CH₃, R⁵ is Cl, R⁶ is 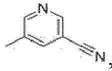 , *n*=3)

Compound XVI-4 (200 g, 0.30 mmol) and glycine methyl ester hydrochloride(148 mg, 1.18 mmol) were used as raw materials, the operation process was the same as that of compound II-1 to obtain 76 mg of brownish solid powder with a yield of 32.3%.

¹H NMR (400 MHz, DMSO-*d*₆) *δ* 8.78 (s, 1H, ArH), 8.61 (s, 1H, ArH), 8.20 (s, 1H, ArH), 8.11 (s, 1H, ArH), 7.67 (d, *J* = 3.6 Hz, 1H, ArH), 7.57 (s, 1H, ArH), 7.56-7.51 (m, 1H, ArH), 7.42 (dd, *J* = 8.5, 4.3 Hz, 2H, ArH), 7.22-7.19 (m, 2H, ArH), 7.09 (d, *J* = 7.4 Hz, 1H, ArH), 5.38 (s, 2H, ArCH₂O), 5.32 (s, 2H, ArCH₂O), 4.61 (t, *J* = 6.5 Hz, 2H, NCH₂), 4.14 (s, 2H, ArCH₂), 4.12-4.08 (m, 2H, OCH₂CH₂), 4.03 (s, 2H, ArCH₂), 3.43 (s, 2H, NHCH₂CO), 3.35 (s, 2H, NHCH₂CO), 2.40-2.30 (m, 2H, CH₂CH₂CH₂).

### Example 18

(5-chloro-2-((5-cyanopyridin-3-)methoxy)-4-((3'-(3-(4-((((R)-1-ethoxy-3-hydroxy-1-oxopropan-2-)ami no)methyl)-1*H*-1,2,3-triazol-1-)propoxy)-2,2'-dimethyl-[1,1'-biphenyl]-3-)methoxy) benzyl)-D-serine ethyl ester (11-18: R¹ and R² are 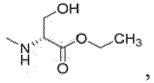 R³ and R⁴ are -CH₃, R⁵ is Cl, R⁶ is 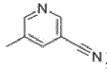 ; *n*=3)

Compound XVI-1 (0.20g g, 0.31 mmol) and triethylamine (0.26 g, 1.89 mmol) and D-serine ethyl ester hydrochloride (0.32 g, 1.89 mmol) were used as raw materials, the operation process was the same as that of compound II-2 to obtain 0.14 g of yellow solid powder with a yield of 46.1 %.

¹H NMR (400 MHz, Chloroform-*d*) *δ* 8.89 (s, 2H, ArH), 8.24 (s, 1H , ArH), 7.54 (s, 1H , ArH), 7.48 (d, *J= 7.5* Hz, 1H , ArH), 7.34 (s, 1H, ArH), 7.30-7.26 (m, 2H, ArH), 7.16 (d, *J* = 7.5 Hz, 1H , ArH), 6.84 (d, *J* = 8.2 Hz, 1H, ArH), 6.79 (d, *J* = 8.2 Hz, 1H, ArH), 6.63 (s, 1H, ArH), 5.20 (s, 2H, ArCH₂O), 5.14 (s, 2H, ArCH₂O), 4.63 (t, *J* = 6.9 Hz, 2H, NCH₂), 4.29-4.14 (m, 4H, OCH₂CH₃), 4.11-4.01(m, 4H, CH₂OH & OCH₂), 3.92-3.86 (m, 2H, CH₂OH), 3.83-3.80 (m, 2H, ArCH₂N), 3.67-3.60 (m, 2H, ArCH₂NH), 3.51 (t, *J* = 5.2 Hz, 1H, CHCO), 3.41 (t, *J* = 5.4 Hz, 1H, CHCO), 2.51-2.34 (m, 2H, CH₂CH₂CH₂), 2.08 (s, 3H, ArCH3), 1.95 (s, 3H, ArCH3), 1.32 - 1.21 (m, 6H, OCH₂CH₃).

HRMS (ESI): *m*/*z* [M+H]⁺, calculated for C₄₅H₅₃ClN₇O₉, 870.3593; found: 870.3589.

### Example 19

(5-chloro-2-((5-cyanopyridin-3-)methoxy)-4-((3'-(3-(4-((((*R*)-1-methoxy-3-hydroxy-1-oxopropan-2-)a mino)methyl)-1*H*-1,2,3-triazol-1-)propoxy)-2,2'-dimethyl- [1,1'-biphenyl]-3-)methoxy)benzyl)-*D*-serine methyl ester (II-19: R¹ and R² are 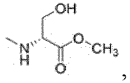 R³ and R⁴ are -CH₃, R⁵ is Cl, R⁶ is 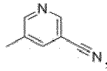 , *n*=3)

Compound XVI-1 (0.28 g, 0.44 mmol), triethylamine (0.24 g, 1.76 mmol) and D-serine methyl ester hydrochloride (274 mg, 1.76 mmol) were used as raw materials, the operation process was the same as that of compound II-2 to obtain 0.12 mg of yellow solid powder with a yield of 32.4 %.

¹H NMR (400 MHz, Chloroform-*d*) *δ* 8.91-8.89 (m, 2H, ArH), 8.26 (s, 1H, ArH), 7.56 (s, 1H, ArH), 7.47 (dd, *J* = 7.6, 1.4 Hz, 1H, ArH), 7.35 (s, 1H, ArH), 7.30-7.26 (m, 1H, ArH), 7.21 (t, *J* = 7.9 Hz, 1H, ArH), 7.16 (dd, *J* = 7.6, 1.4 Hz, 1H, ArH), 6.83 (d, *J* = 8.2 Hz, 1H, ArH), 6.79 (dd, *J* = 7.6, 1.1 Hz, 1H, ArH), 6.64 (s, 1H, ArH), 5.19 (s, 2H, ArCH₂O), 5.16 (s, 2H, ArCH₂O), 4.64 (t, 2H, *J*= 4.0 Hz, NCH₂), 4.10-4.02 (m, 4H, CH₂OH & OCH₂), 3.92-3.87 (m, 2H, CH₂OH), 3.83-3.80 (m, 2H, ArCH₂NH), 3.76 (s, 3H, OCH₃), 3.73 (s, 3H, OCH₃), 3.70-3.63 (m, 2H, ArCH₂NH), 3.54 (t, *J*= 5.1 Hz, 1H, CHCO), 3.46 (t, *J*= 6.1 Hz, 1H, CHCO), 2.51-2.43 (m, 2H, CH₂CH₂CH₂), 2.11 (s, 3H, ArCH₃), 1.95 (s, 3H, ArCH₃).

HRMS (ESI): *m*/*z* [M+H]⁺, calculated for C₄₃H₄₉ClN₇O₉, 842.3280; found: 842.3261.

### Example 20

(5-chloro-2-((5-cyanopyridin-3-yl)methoxy)-4-((3'-(3-(4-(((2-ethoxy-2-oxo ethyl)amino)methyl)-1*H*-1,2,3-triazol-1-)propoxy)-2,2'-dimethyl-[1,1'-biphenyl]-3-)methoxy)benzyl)glycine ethyl ester (II-20: R¹ and R² are 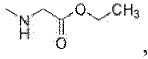 R³ and R⁴ are -CH₃, R⁵ is Cl, R⁶ is 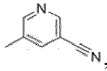 , *n*=3)

Compound XVI-1 (0.20g g, 0.31 mmol), glycine ethyl ester hydrochloride (0.26 g, 1.89 mmol) and triethylamine (0.26 mL, 1.89 mmol) were used as raw materials, the operation process was the same as that of compound II-2 to obtain 0.11 g of a yellow viscous product with a yield of 40.1 %.

¹H NMR (300 MHz, Chloroform-*d*) *δ* 8.97 (s, 2H, ArH), 8.32 (s, 1H, ArH), 7.63 (s, 1H, ArH), 7.55 (d, *J* = 7.6 Hz, 1H, ArH), 7.45 (s, 1H, ArH), 7.38 (m, 1H, ArH), 7.29 (d, *J* = 7.9 Hz, 1H, ArH), 7.25-7.21 (m, 1H, ArH), 6.90 (d, *J* = 8.2 Hz, 1H, ArH), 6.86 (d, *J* = 7.5 Hz, 1H, ArH), 6.72 (s, 1H, ArH), 5.26 (s, 2H, ArCH₂O), 5.22 (s, 2H, ArCH₂O), 4.71 (t, *J* = 6.9 Hz, 2H, NCH₂), 4.27 (q, *J* = 7.1 Hz, 4H, CH₂CH₃), 4.13 (q, *J* = 5.3, 4.7 Hz, 2H, OCH₂), 4.04 (s, 2H, ArCH₂NH), 3.87 (s, 2H ArCH₂NH), 3.55 (s, 2H, NHCH₂CO), 3.51 (s, 2H, NHCH₂CO), 2.54 (q, *J* = 6.4 Hz, 2H, CH₂CH₂CH₂), 2.19 (s, 3H, ArCH₃), 2.03 (s, 3H, ArCH₃), 1.36 (t, 3H, *J* = 7.0 Hz, ArCH₃), 1.35 (t, 3H, *J* = 7.2 Hz, ArCH₃).

HRMS (ESI): *m*/*z* [M+H]⁺, calculated for C₄₃H₄₉ClN₇O₇, 840.3382; found: 840.3363.

### Example 21

((1-(3-((3'-((2-chloro-4-((((R)1-ethoxy-3-hydroxy-1-oxopropan-2-)amino)methyl)-5-methoxyphenoxy) methyl)-2,2'-dimethyl-[1,1'-biphenyl]-3-)oxy)propyl)-]-1*H*-1,2,3-triazol-4-)methyl)-D-serine ethyl ester (II-21: R¹ and R² are 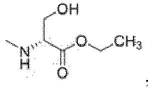 , R³ and R⁴ are -CH₃, R⁵ is Cl, R⁶ is H, *n*=3)

Compound XVI-3 (0.20g g, 0.37 mmol), D-serine ethyl ester hydrochloride (0.38 g, 2.22 mmol) and triethylamine (0.31 mL, 2.22 mmol) were used as raw materials, the operation process was the same as that of compound II-2 to obtain 0.16 g of a light yellow viscous product with a yield of 57.1 %.

¹H NMR (300 MHz, Chloroform-*d*) *δ* 7.58 (s, 1H, ArH), 7.53 (d, *J* = 7.3 Hz, 1H, ArH), 7.33 (s, 1H, ArH), 7.28 (s, 1H, ArH), 7.23 (t, *J* = 7.9 Hz, 1H, ArH), 7.19-7.15 (m, 1H, ArH), 6.84 (t, *J* = 8.8 Hz, 2H, ArH), 6.62 (s, 1H, ArH), 5.22 (s, 2H, ArCH₂O), 4.66 (t, *J* = 6.8 Hz, 2H, NCH₂), 4.28 (q, *J* = 7.1 Hz, 2H, OCH₂CH₃), 4.20 (q, *J* = 7.1 Hz, 2H, OCH₂CH₃), 4.18-4.02 (m, 4H, CH₂OH & OCH₂), 3.96-3.91 (m, 2H, CH₂OH), 3.88 (s, 3H, OCH₃), 3.84-3.80 (m, 2H, ArCH₂NH), 3.76-3.66 (m, 4H, CH₂CH₃), 3.56-3.53 (m, 1H, CHCO), 3.45 (t, *J =* 5.2 Hz, 1H, CHCO), 2.51 (d, *J* = 6.2 Hz, 2H, CH₂CH₂CH₂), 2.14 (s, 3H, ArCH₃), 1.98 (s, 3H, ArCH₃), 1.30 (t, *J* = 7.4 Hz, 6H, CH₂CH₃).

HRMS (ESI): *m*/*z* [M+H]⁺, calculated for C₃₉H₅₁ClN₅O₉, 768.3375; found: 768.3360.

### Example 22

Pharmacological trials and results of some compounds of the invention are as follows:
1. Evaluation of the inhibitory activity of compounds of the invention on the interaction of hPD-1/hPD-L1

Purpose of experiment: The hPD-1/hPD-L1 binding assay kit (CISBIO company) is used to detect the inhibitory activity of the compounds of the invention on PD-1/PD-L1.

Experimental principle: Homogeneous Time-Resolved Fluorescence (HTRF) is a technique used to detect analytes in pure liquid phase systems. It mainly uses the energy transfer of two fluorescent groups, which are divided into an energy donor europium (Eu+) and an energy acceptor. When the donor is externally excited (such as by a flash lamp or laser), the energy resonance can be transferred to the receptor if it is within a close enough distance to the receptor, and the receptor is excited to emit a specific wavelength of light. Using HTRF technology, the assay enables simple and rapid characterization of compounds and antibody blockers in a high-throughput format. By using anti-Tag1 tagged with Eu+ (europium) (HTRF energy donor) and anti-Tag2 tagged with XL665 (HTRF energy acceptor), the interaction between PD-L1 and PD-1 can be detected. Tag1 and Tag2 are used to mark the PD-L1 protein and PD-1 protein, respectively, and Eu⁺ and XL665 are bound to PD-L1 and PD-1, respectively, by antibodies to form complexes. When PD-L1 and PD-1 are bound in close proximity to each other, Eu⁺ is excited by an external laser to trigger fluorescence resonance energy transfer towards XL665, which in turn emits specifically at 665 nm. This specific signal is directly proportional to the degree of PD1/PD-L1 interaction. Therefore, compounds or antibodies that block the PD-1/PD-L1 interaction will result in decreased HTRF signaling.

Trial Materials: The kit was purchased as the hPD-1/hPD-L1 binding assay kits from CISBIO; 96-well plate: purchased from CISBIO Corporation.

Test instruments: Perkin Elmer, model: EnVision.

Test compounds: The invention prepares the obtained compounds II-1 to II-21. DMSO was used to dissolve and the diluent buffer was used to dilute; the DMSO concentration should not exceed 0.5%.

Experimental procedure: hPD-1/hPD-L1 binding assay kits was used. A negative control, a positive control and drug administration groups were set up, with two duplicate wells in each group. For the positive control group, added 2 µL diluent to a 96-well plate; 4 µL of PD-L1 and 4 uL of PD-1 as diluted according to the instructions; for the negative control group, added 6 µL diluent and 4 µL PD-L1 to a 96-well plate; for the administration group, 2 µL of the compound of the invention (or the positive compound BMS-202), 4 µL of PD-L1 and 4 µL of PD-1 were successively added to a 96-well plate. Sealed the plate with a sealing film, centrifuged at 1000 rpm for 1 minute, and incubated at room temperature for 15 minutes. Then mixed equal volumes of Anti-Tag-Eu3⁺ and Anti-tag-XI,665 as diluted in buffer evenly, then added 10 µL of the mixture to each well, sealed the plate, centrifuged at 1000 rpm for 1 minute, and incubated at room temperature for 2 hours. Removed the sealing film, used EnVision to read the fluorescence intensity at 665 nm and 615 nm, and calculate ratio=Signal 665 nm/ Signal 620 nm × 10⁴. IC₅₀ of the compounds was calculated using Graphpad. In this experiment, BMS-202 in the patent WO2015034820 of BMS Company was selected as the positive drug, and the activity data is shown in Table 1.

A indicates 0.10-10 nM; B indicates 10.01-100 nM; C indicates 100.01 nM-1 µM.

**Table 1 Inhibitory activity of compounds on hPD-1/hPD-L1 interactions**

| Compound number | PD-1/PD-L1 IC₅₀(nM) | Compound number | PD-1/PD-L1 IC₅₀(nM) |
|---|---|---|---|
| II-1 | A | II-9 | A |
| II-2 | B | II-10 | B |
| II-3 | B | II-11 | B |
| II-4 | B | II-12 | A |
| II-5 | A | II-13 | A |
| II-6 | C | II-14 | B |
| II-7 | A | II-15 | A |
| II-8 | B | II-16 | B |
| II-17 | C | II-18 | B |
| II-19 | B | II-20 | B |
| II-21 | B | BMS-202 | B |

The trial results show that the compound of the invention has significant PD-1/PD-L1 inhibitory activity. It is predicted that the biphenyl compounds of the invention can be used to prepare PD-1/PD-L1 inhibitor anti-tumor drugs.

### 2. Affinity test between the compounds of the invention and hPD-L1 protein

Experimental principle: When the incident light was imposed on the interface of two media with different refractive indices (such as gold or silver coating on a glass surface) at a critical angle, it could cause the resonance of the free electrons of the metal. Due to the resonance, the electrons absorbed light energy, so that the reflected light was greatly weakened at certain angles. Among them, the incident angle at which the reflected light completely disappeared within a certain angle was called the surface plasmon resonance (SPR) angle. The SPR angle varied with the surface refractive index, which in turn varied in proportional to the mass of biomolecules bound to the metal surface. The reversible association/dissociation between molecules caused real-time changes in the refractive index near the gold film, which was recorded by Biacore in real time.

### Experimental reagents:

Fc-hPD-L1 protein was purchased from Aero Biosystems, and PBS-P+ buffer 10x buffer, 10 mM glycine pH 1.5 and Series S Sensor Chip Protein A chips were purchased from GE Healthcare Bio-sciences.

### Experimental methods:

The test compound was dissolved in DMSO to make a stock solution with a final concentration of 10mM, then diluted to 1x with 1.05x PBS-P+, and then diluted by 4 times with 1x PBS-P+ buffer containing 5% DMSO; the Fc-PD-L1 protein was diluted to 10 µg/mL with 1x PBS-P+ solution containing 5% DMSO, and was then captured on the Protein A chip at a flow rate of 10 µl/min for 120s. Each experiment used dual channels, one of which captured the ligand and the other served as a reference channel; the analyte was ready for use, and at least five concentrations were set for each analyte. The concentration selection varied with the analyte. The analyte flowed through the two channels at 30 µl/min for 90 seconds; solvent calibration was used for exclusion of the influence of DMSO, 1x PBS-P+ in 50% DMSO solution was used to wash the flow path with 30 µl/min; 10 mM glycine pH 1.5 for 30s was used for chip surface regeneration, the experimental temperature is 25 °C; Biacore T200 Evaluation software was used, 1: 1 binding model was used to calculate the KD value. The data are shown in Table 2. Note: A indicates 0.10-100 nM; B indicates 100.01-1000 nM.

**Table 2. Compounds and hPD-L1 affinity values**

| Compound | KD(nM) | Compound | KD(nM) |
|---|---|---|---|
| II-1 | A | II-8 | A |
| II-3 | A | II-9 | A |
| II-5 | A | II-11 | A |
| II-7 | A | II-12 | A |
| II-18 | B | II-19 | B |
| II-20 | B | II-21 | B |

The experimental results show that all the compounds of the invention can bind to PD-L1 protein with very high affinity, which indicates that the biphenyl compounds of the invention have strong inhibitory activity on PD-L1 protein.

### 3. The experiment in which the compounds of the invention block PD-L1 to inhibit the expression of INF-γ secreted by T cells

Experimental principle: Hep3B-OS8-hPDL1 cells (Shanghai Ruizhi Chemical Research Co., Ltd.) stably express hPD-L1 protein on the surface; CD3+ T cells (Shanghai Ruizhi Chemical Research Co., Ltd.) express PD-1 on the surface; when the two cell lines are co-cultured, hPD-L1 on the surface of Hep3B-OS8-hPDL1 cells will interact with the protein PD-1 protein on the surface of CD3+T cells, thereby inhibiting the activation, proliferation and expression of the immune factor INF-γ of CD3+ T cells. When the compound blocks the PD-1/PD-L1 interaction, it will relieve the inhibition of CD3+ T cells, thereby promoting the expression of INF-γ.

Experimental procedure: Whole blood was stored in EDTA anticoagulant tubes, and PBMCs were separated by density gradient centrifugation. CD3+T cells were further isolated from PBMCs with the EasySepTM Human T Cell Isolation Kit and the cells were resuspended in RPMI-1640 complete culture medium to adjust the concentration to 5×10⁵/mL. Hep3B-OS8-hPDL1 cells were treated with 10 µg/mL mitomycin, then incubated at 37°C for 1.5 h, washed four times with PBS, and resuspended in complete RPMI-1640 medium to adjust the concentration to 5×10⁵/mL. Added Hep3B-OS8-hPDL1 (50 µL/well) and T cells (100 µL/well) into a 96-well round-bottom microplate. Prepared 4×pembrolizumab (50 µL/well) and 4 × test compound (50 µL/well) with RPMI-1640 complete culture medium, and added the prepared compound and pembrolizumab to the corresponding wells (the final concentration of pembrolizumab was 5 µg/mL), with a total volume of 200 µL. Three concentration gradients were set for each drug with duplicate wells and pembrolizumab and BMS-202 were used as the positive control group. Incubated for 72 hours in an incubator at 37°C with 5% CO₂. After centrifugation at 350 × g for 5 minutes, 150 µL of the supernatant was obtained, and the secretion of IFN-γ is detected by ELISA. Data processing was performed using GraphPad Prism6.

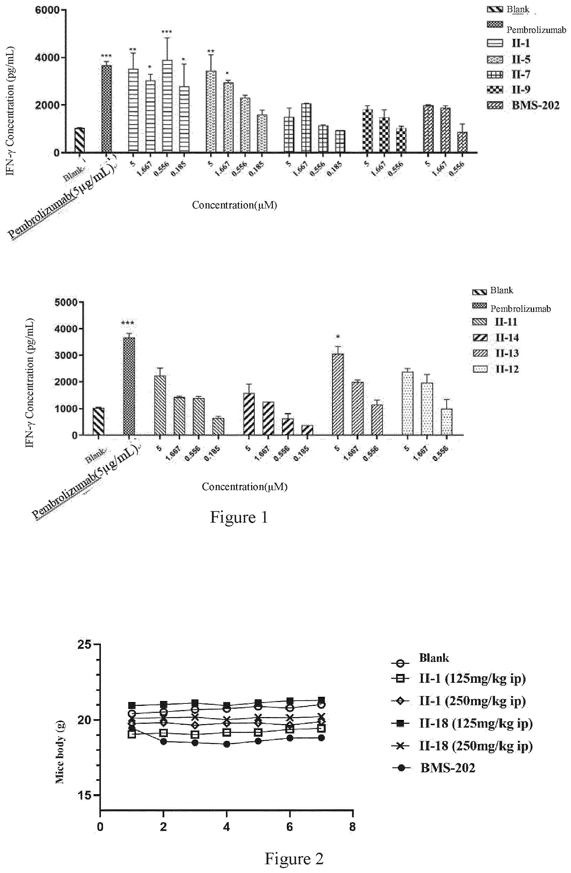

The result is shown in Figure 1. Remarks: Significant differences were calculated using one-way analysis of variance (ANOVA); **P* < 0.05; ***P* < 0.01; ****P* < 0.001 vs Blank. From the experimental results, it can be clearly seen that all the compounds of the invention can block the PD-1/PD-L1 interaction, thereby releasing the inhibitory effect on CD3+T cells and significantly promoting the expression of INF-γ. The compounds of the invention dose-dependently promoted the expression of INF-γ, and the effect was significantly higher than that of BMS-202, slightly lower than the effect of pembrolizumab (5 µg/mL), and thus they had the effect of enhancing the anti-tumor effect of T cells; therefore, the biphenyl compounds of the invention can be used as immune checkpoint PD-1/PD-L1 inhibitors to prepare a drug for tumor immunotherapy.

### 4. Acute Toxicity Test of the Compounds of the Invention

Purpose of experiment: In order to obtain a safe dosage of the compounds, we administered intraperitoneal injection of compounds 11-1 and 11-18 (250 mg/kg and 125 mg/kg) and BMS-202 (125 mg/kg) respectively, and observed the state of mice.

Experimental procedure: C57BL/6 mice were used and kept in a quarantine room for 3 days. They were randomly divided into five groups with five animals in each group; the drug was administered by intraperitoneal injection (0.2 mL/20 g), the state of the animals was observed, and the body weight was measured.

Experimental results: As shown in Figure 2, after intraperitoneal injection, the mice in the compounds **II-1** and **II-18** (250 mg/kg and 125 mg/kg) groups did not die, nor did their body weight decrease. After intraperitoneal injection of BMS-202 (125 mg/kg), the mice showed obvious signs of convulsions and tremors, and the body weight of the mice decreased significantly. Therefore, the compound of the invention showed higher safety when compared with BMS-202.

### 5. Anti-tumor Effects of Compounds of the Invention in Mice

Purpose of experiment: In order to accurately evaluate the anti-tumor effect of the compound in vivo, we used the MC38-hPDL1 cell xenograft model of humanized C57BL/6 mice (Shanghai Southern Model Biotechnology Co., Ltd.) to evaluate the anti-tumor effect of the compounds

Experimental procedure: Colon cancer cells MC38-hPDL1 were cultured in an incubator at 37°C and 5% CO₂, and the medium was DMEM medium containing 10% inactivated fetal bovine serum. The cells were sub-cultured and passaged every 3 to 4 days when confluent.

Take the cells in the logarithmic growth phase, resuspend them in PBS, count the cells, and adjust the cell concentration to 1.0 × 10⁷/mL; use a 1 mL syringe to inoculate the cell suspension on the right flank of C57BL/6J-hPDL1 mice, 100 µL/mouse, subcutaneous injection of tumor cells (about 1.0×10⁶/mouse). Observe the mice daily until the average tumor volume is around 40-60 mm³.

After successful modeling, the mice were kept in a quarantine room for 3 days and raised in an environment facility for SPF grade experimental animals. The size of the xenografted tumor in mice was measured with a vernier caliper, and the mice that met the experimental requirements were selected and randomly divided into four groups, with five mice in each cage, and the average tumor volume in each group was about 54 mm³. II-1 (intraperitoneal injection, 25 mg/kg) or II-1 (intragastric administration, 25 mg/kg) and II-18 (intragastric administration, 25 mg/kg) was given twice a day; body weight change and tumor volume were measured every two days. Tumor volume (TV) is calculated using the formula: (TV=1/2 × length × width × width). After 14 days of administration, the mice were sacrificed, the tumor tissues were weighed and photographed, and the data were processed with graphpad prism. Each mouse's spleen was taken, ground and processed, and the proliferation of CD3⁺CD4⁺CD8⁺T cells was measured by flow cytometry. The calculation formula is as follows: Relative tumor proliferation rate T/C % = T_{RTV}/C_{RTV}*100%. (T_{RTV}: RTV for treatment group; C_{RTV}: RTV for negative control group).

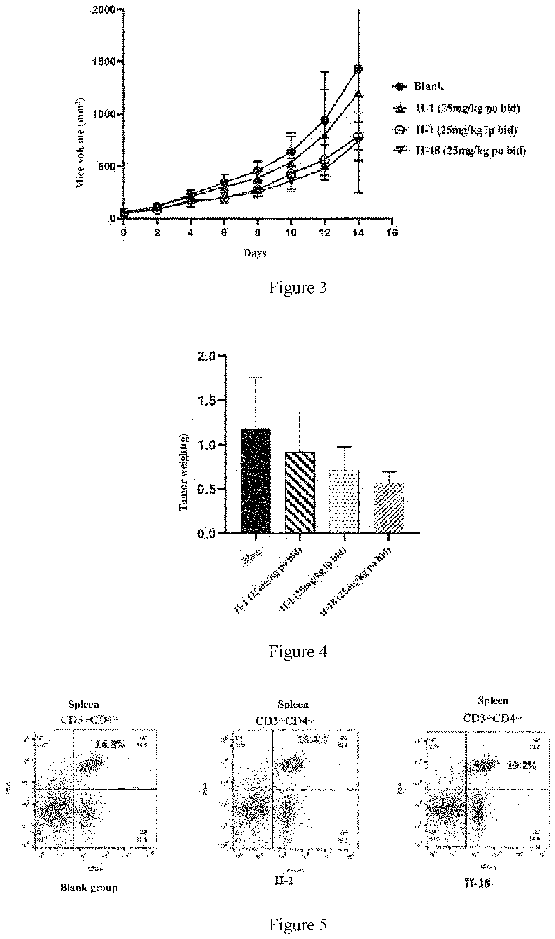

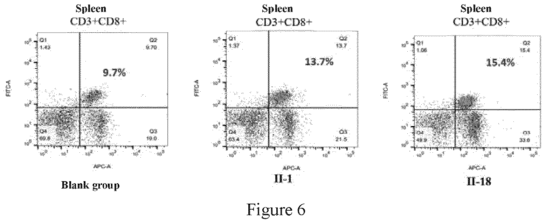

Experimental results: Compared with the model blank group, compounds 11-1 (intraperitoneal injection group) and 11-18 (gastric administration group) can significantly inhibit the growth of colon cancer (relative tumor inhibition rates were 45.8% and 49.6%; Figure 3), while compound II-1 (intragastric administration group) has a weak inhibitory effect on tumor growth. By comparing the chemical structures of compounds II-1 and 11-18, it can be found that the two carboxyl groups in the molecule of II-1 are esterified to obtain the prodrug type II-18, and its anti-tumor effect by oral administration is obviously improved. The tumor weight also reflects the excellent anti-tumor effect of compounds II-1 (intraperitoneal administration group) and II-18 (gastric administration group) (**p*<0.05) as shown in Figure 4. The applied compounds II-1 (intraperitoneal injection group) and II-18 (intragastric administration group) can promote the proliferation of CD3⁺CD4⁺T cell and CD3⁺CD8⁺T cell classifications as shown in Figures 5 and 6, respectively.

## Claims

1. A compound represented by formula I or pharmaceutically acceptable salt thereof: 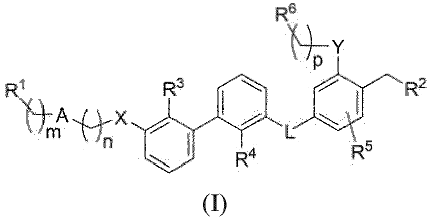 wherein
X and Y independently represent O, NH, S or CH₂; *m*=0, 1 or 2; *n*=1, 2, 3 or 4; *p*=0 or 1;
A represents a substituted phenyl or aromatic heterocyclic group, the aromatic heterocyclic group is a five- or six-membered aromatic ring containing 1 to 3 O atoms, N or S atoms, and the substituents are H, F, Cl, Br, CN, NH₂, OH, CF₃, OCF₃, C₁-C₃ alkyl or C₁-C₃ alkoxy;
L represents -CH₂O-, -CH₂NH-, -CONH-, -NHCO-, -OCH₂-, -NH- or -CH=CH-;
R¹ and R² each independently represents NR⁷R⁸, OR⁸ or a substituted C₄-C₆ azacycloalkyl, wherein R⁷ represents H or C₁-C₃ alkyl; R⁸ represents a substituted C₁-C₆ alkyl, and the substituents are OH, NH₂, COOH, an amide group, an ester group, an alkoxy group, which can be mono- or poly-substituted; the substituted C₄-C₆ azacycloalkyl group is a substituted tetrahydropyrrol-1-yl, piperidin-1-yl, morpholin-1-yl, piperazin-1-yl or azetidin-1-yl, wherein the substituents are OH, NH₂, COOH, an amide group, an ester group and/or an alkoxy group, which can be mono- or poly-substituted;
R³ and R⁴ each independently represents H, F, Cl, Br, CN, CF₃, C₁-C₃ alkyl or cyclopropyl;
R⁵ represents H, F, Cl, Br, CN, NH₂,OH, CF₃, OCF₃, C₁-C₃ alkyl or C₁-C₃ alkoxy; and
R⁶ represents H, C₁-C₃ alkyl, C₁-C₃ haloalkyl, C₃-C₆ cycloalkyl, substituted phenyl or substituted aromatic heterocyclic group, and the aromatic heterocyclic group as specified contains the five-membered or six-membered aromatic ring with 1-3 O, N or S atoms, and the substituents are H, F, Cl, Br, CN, amido, sulfonamide, methanesulfonyl and/or trifluoromethyl.

2. The compound or pharmaceutically acceptable salt thereof according to claim 1, wherein X and Y represent O; *m*=1, *n*=2 or 3; *p*=1; A represents 1,4- substituted 1,2,3-triazole ring; and L represents -CH₂O-.

3. The compound or pharmaceutically acceptable salt thereof according to claim 1, wherein R¹ and R² each independently represents: OH, 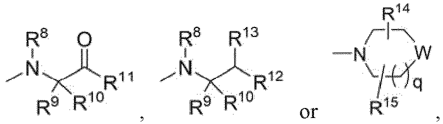 wherein R⁸ represents CH₃, CH₂CH₃, CH₂CH₂OH, formyl, acetyl, cyclopropyl; R⁹ and R¹⁰ each represent H, OH, COOH, CH₂COOH, CH₂NH₂, CH₂OH, CH₂CH₂OH, F, Cl, Br, CH₃, CH₂CH₃, and/or cyclopropyl; R¹¹ represents OH, NH₂, NHCH₃, NHCH₂CH₃, CH₃, OCH₃, OCH₂CH₃, OC(CH₃)₃, and/or OCH(CH₃)₂, 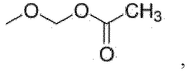 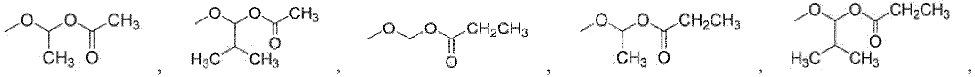 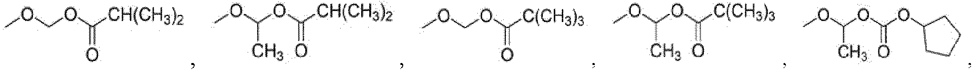 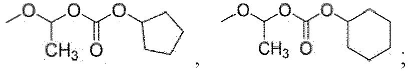 R¹² represents CONH₂, NHCOCH₃, OH, CH₂OH, CH₂CH₂OH, COOH, 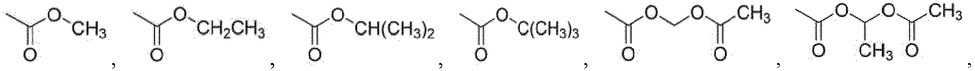 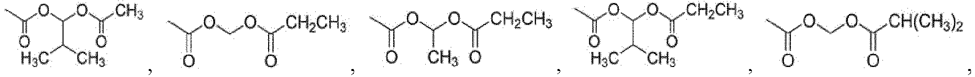 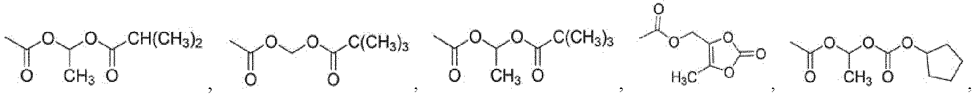 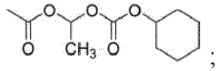 R¹³ represents H, CH₃, CH₂CH₃, CH₂OH, and/or CH₂CH₂OH; R¹⁴ and R¹⁵ each represent H, COOH, NH₂, F, Cl, Br, CH₃, CH₂CH₃, CH₂OH, CH₂CH₂OH, CONH₂, and/or cyclopropyl, 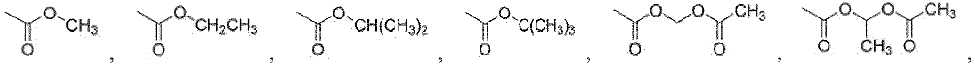 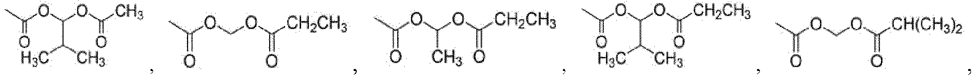 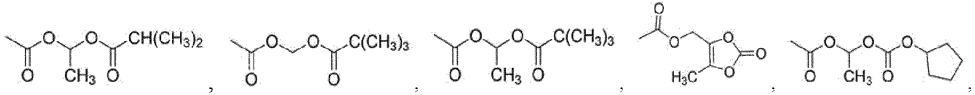 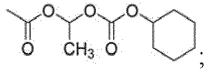 W represents CH₂, O, NH, N-CH₃, N-CH₂CH₃, N-CH₂CH₂OH, and/or N-COCH₃; and q represents 0 or 1.

4. The compound or pharmaceutically acceptable salt thereof according to claim 1, wherein R³ and R⁴ each independently represents F, Cl, Br, CN or CH₃; R⁵ represents H, F, Cl, Br, CH₃ or OCH₃; and R⁶ represents H or 5-cyanopyridin-3-yl.

5. The compounds or pharmaceutically acceptable salt thereof according to claim 1, wherein R¹ and R² each independently represents: 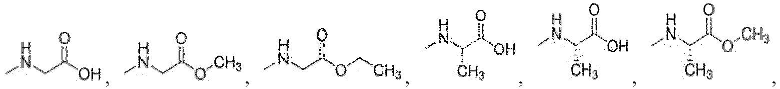 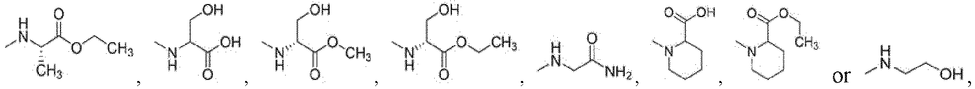 R³ and R⁴ represent CH₃, R⁵ represents Cl, and R⁶ represents H or 5-cyanopyridin-3-yl.

6. The compound or pharmaceutically acceptable salt thereof according to any one of claims 1 to 5, wherein the pharmaceutically acceptable salt is an acid addition salt formed by the compound of general formula (I) according to claim 1 and the following acids: hydrogen chloride, hydrogen bromide, sulfuric acid, carbonic acid, oxalic acid, citric acid, succinic acid, tartaric acid, phosphoric acid, lactic acid, pyruvic acid, acetic acid, maleic acid, methanesulfonic acid, benzenesulfonic acid, p-toluenesulfonic acid, or ferulic acid.

7. The compound or pharmaceutically acceptable salt thereof according to claim 1, wherein the compound is II-1, II-2, II-3, II-4, II-5, II-7, II-8, II-9, II-10, II-11, II-12, II-13, II-14, II-15, II-16, II-18, II-19, II-20, or II-21.

8. A preparation method for the compound according to claim 2, wherein a synthetic route of the preparation method is as follows:

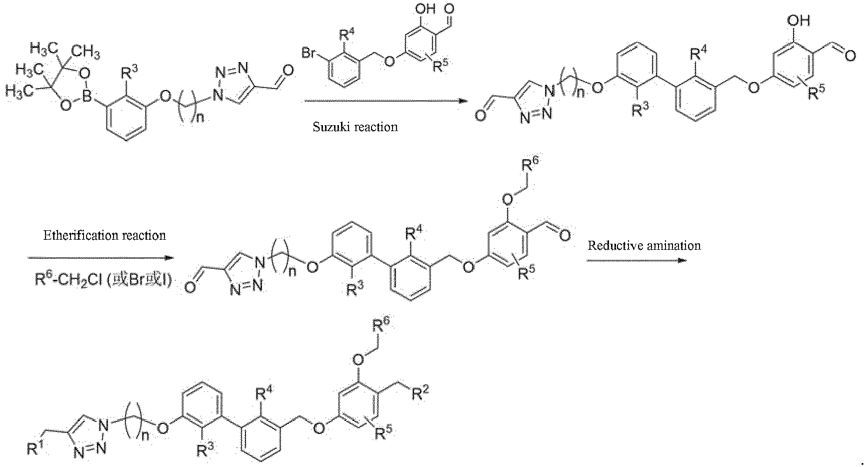

9. A pharmaceutical composition, comprising the compound or pharmaceutically acceptable salt thereof according to any one of claims 1 to 7 and a pharmaceutically acceptable carrier.

10. A use of the compound or pharmaceutically acceptable salt thereof according to any one of claims 1 to 7 in preparation of a PD-1/PD-L1 inhibitor.

11. A use of the compound or pharmaceutically acceptable salt thereof according to any one of claims 1 to 7 in preparation of a medicament for antitumor.
